# EUROPÄISCHE PATENTSCHRIFT

(11) **EP 2 598 435 B1**
(45) Veröffentlichungstag und Bekanntmachung des Hinweises auf die Patenterteilung: **01.10.2014**
(21) Anmeldenummer: 11740561.3
(22) Anmeldetag: 15.07.2011
(51) Int. Cl.: A61K 8/22, A61K 8/49, A61Q 5/04, A61Q 5/08, A61Q 5/10, A61Q 11/00, A61K 8/81, C11D 3/28, C11D 3/37, C11D 7/32, C01B 15/037, C01B 15/08, C01B 15/12, C11D 3/39

(54) **ZUSAMMENSETZUNGEN ENTHALTEND WASSERSTOFFPEROXID ODER WASSERSTOFFPEROXID FREISETZENDE SUBSTANZEN**
COMPOSITIONS COMPRISING HYDROGEN PEROXIDE OR HYDROGEN PEROXIDE DONOR SUBSTANCES
COMPOSITIONS CONTENANT DU PEROXYDE D'HYDROGÈNE OU SUBSTANCES LIBÉRANT DU PEROXYDE D'HYDROGÈNE

(30) Priorität: 17.12.2010 DE 102010054918; 27.07.2010 DE 102010032371
(43) Veröffentlichungstag der Anmeldung: 05.06.2013
(73) Patentinhaber: Clariant Finance (BVI) Limited, Road Town, Tortola (VG)
(72) Erfinder: KLUG, Peter, 63762 Großostheim (DE); PILZ, Maurice, Frederic, 60329 Frankfurt am Main (DE); BACK, Ute, 63825 Blankenbach (DE)
(74) Vertreter: Paczkowski, Marcus
(86) Internationale Anmeldenummer: PCT/EP2011/003536
(87) Internationale Veröffentlichungsnummer: WO 2012/019688

(56) Entgegenhaltungen:
- EP-A1- 1 347 736
- WO-A2-02/051961
- WO-A2-2009/015856
- FR-A1- 2 804 863
- US-A- 6 083 422
- US-A1- 2006 009 371

## Beschreibung

Die vorliegende Erfindung betrifft Zusammensetzungen enthaltend Wasserstoffperoxid oder Wasserstoffperoxid freisetzende Substanzen.

Wasserhaltige Zusammensetzungen enthaltend Wasserstoffperoxid werden in verschiedenen Anwendungen benutzt. Sie werden in kosmetischen Mitteln z. B. als Bleichzusammensetzung für Haar, als Entwicklerkomponente in Haarfärbemitteln, aber auch als Komponente zur Haarfixierung in Dauerwellformulierungen verwendet. Weitere Anwendungen sind z. B. Zahnbleichzusammensetzungen. Auch in der industriellen Reinigung und in der Haushaltsreinigung sind saure, wasserstoffperoxidhaltige Zusammensetzungen in Reinigerformulierungen vertreten.

Aufgrund der niedrigen Viskosität von wässrigen Wasserstoffperoxidlösungen, sind wässrige Lösungen nicht immer vorteilhaft, da sie aufgrund der niedrigen Viskosität nicht punktgenau applizierbar sind, spritzen können oder zu schnell verlaufen.
Auch bei Zugabe von Polyacrylatverdickern kann wegen der Protonierung des Verdickerpolymers bei saurem pH-Wert keine Verdickung erzielt werden (Karlheinz Schrader, Andreas Domsch, Cosmetology - Theory and Practize, Volume II, Verlag für chemische Industrie, Augsburg, 2005, Seiten 114-115).

In der Kosmetik werden deshalb bis heute für Haarfärbeformulierungen vor allem O/W-Emulsionen enthaltend Wasserstoffperoxid benutzt (Karlheinz Schrader, Andreas Domsch, Cosmetology - Theory and Practize, Volume II, Verlag für chemische Industrie, Augsburg, 2005, Seite 145), die aufgrund der höheren Viskosität der Emulsionen besser applizierbar sind. Die Viskosität wird hierbei durch die micellare Struktur der Emulsion erzeugt.

Polymerverdickte Zusammensetzungen enthaltend Wasserstoffperoxid sind literaturbekannt (siehe z. B. US 4,804,705 oder EP 0 829 258). In EP 0 829 258 werden verdickte Wasserstoffperoxidformulierungen beschrieben, die durch die Verwendung von Poly(acryloyldimethyltaurat)polymeren erhöhte Stabilität aufweisen.

EP 1 347 736 beschreibt oxidative Zusammensetzungen zur Haarbehandlung, die ein amphiphiles Polymer auf Basis eines sulfonierten Polymers enthaltend eine hydrophobe Gruppe enthält. Dabei werden Stabilisatoren auf Basis Pyrophosphat, Stannaten, Phenacetin oder Oxychinolin oder deren Kombinationen verwendet.

US 2006/009371 offenbart die Verwendung eines Gemisches bestehend aus einem Stannatstabilisator, einem Phosphor enthaltenden Stabilisator und einem aromatischen Chelator zur Stabilisierung eines oder mehrerer Polymere mit verdickenden Eigenschaften in einer Wasserstoffperoxid und Wasser enthaltenden Zusammensetzung.

US 6 083 422 offenbart die Verwendung eines Stabilisators gemäß der Formel wie offenbart in Anspruch 1 zur Stabilisierung eines oder mehrerer Polymere mit verdickenden Eigenschaften in einer Wasserstoffperoxid und Wasser enthaltenden Zusammensetzung.

Für eine kommerzielle Verwendung sind die obigen Technologien jedoch trotz verbesserter Stabilität bis heute nicht ausreichend lagerstabil. Es stellt sich in der Praxis heraus, dass auch bei Lagerung bei erhöhter Temperatur zwar der Gehalt an Wasserstoffperoxid in den Formulierungen nicht abnimmt, nichtsdestotrotz aber die Viskosität der Formulierung innerhalb von Wochen oft drastisch nachlässt.

Es bestand deshalb ein Bedarf an langfristig lagerstabilen, polymerverdickten Zusammensetzungen enthaltend Wasserstoffperoxid und/oder Wasserstoffperoxid freisetzende Substanzen.

Überraschend wurde nun gefunden, dass durch Zugabe geringer Mengen von Hydroxypyridonen oder deren Salzen die Lagerstabilität wässriger polymerverdickter Zusammensetzungen enthaltend eine oder mehrere Substanzen ausgewählt aus der Gruppe bestehend aus Wasserstoffperoxid und Wasserstoffperoxid freisetzenden Substanzen signifikant verbessert werden kann.

Gegenstand der Erfindung sind somit Zusammensetzungen enthaltend
a) eine oder mehrere Substanzen ausgewählt aus der Gruppe bestehend aus Wasserstoffperoxid und Wasserstoffperoxid freisetzenden Substanzen,
b) Wasser,
c) ein oder mehrere Polymere mit verdickenden Eigenschaften und
d) eine oder mehrere Substanzen ausgewählt aus der Gruppe bestehend aus Hydroxypyridonen und deren Salzen.

Damit sind beispielsweise gelförmige Zusammensetzungen gegeben, die aufgrund der Gegenwart des polymeren Verdickungsmittels die oben genannten Nachteile nicht mehr aufweisen und exzellent appliziert werden können. Insbesondere Zusammensetzungen mit scherverdünnenden Eigenschaften lassen sich sehr gut applizieren.
Hierdurch kann bei der Verwendung der Wasserstoffperoxidformulierungen als Komponente in alkalischen Haarfärbeformulierungen sowohl die Durchmischung der Komponenten als auch die Viskosität nach dem Mischen von Entwickler und Kuppler besser gesteuert werden. Zudem kann auch über die Steuerung der Viskosität die Flüchtigkeit des Ammoniaks und deshalb auch die Geschwindigkeit des Färbeprozesses besser gesteuert werden.

Vorzugsweise ist die eine oder sind die mehreren Substanzen der Komponente a) ausgewählt aus der Gruppe bestehend aus Wasserstoffperoxid, Harnstoffperoxid, Perboraten, Persulfaten und Mischungen davon. Besonders bevorzugt ist die Substanz der Komponente a) Wasserstoffperoxid.

Die eine oder die mehreren Substanzen ausgewählt aus der Gruppe bestehend aus Wasserstoffperoxid und Wasserstoffperoxid freisetzenden Substanzen der Komponente a) sind vorzugsweise in Mengen von 0,5 bis 20 Gew.-%, besonders bevorzugt in Mengen von 1 bis 10 Gew.-%, insbesondere bevorzugt in Mengen von 1,5 bis 7 Gew.-% und außerordentlich bevorzugt in Mengen von 2 bis 7 Gew.-% in den erfindungsgemäßen Zusammensetzungen enthalten, bezogen auf das Gesamtgewicht der Zusammensetzungen. Hierunter wiederum bevorzugt ist die Substanz der Komponente a) Wasserstoffperoxid, welches vorzugsweise in Mengen von 0,5 bis 20 Gew.-%, besonders bevorzugt in Mengen von 1 bis 10 Gew.-%, insbesondere bevorzugt in Mengen von 1,5 bis 7 Gew.-% und außerordentlich bevorzugt in Mengen von 2 bis 7 Gew.-% in den erfindungsgemäßen Zusammensetzungen enthalten ist, bezogen auf das Gesamtgewicht der Zusammensetzungen.

In einer bevorzugten Ausführungsform der Erfindung ist das Wasser (Komponente b)) in einer Menge von 40 Gew.-% oder mehr und vorzugsweise in einer Menge von 50 Gew.-% oder mehr in den erfindungsgemäßen Zusammensetzungen enthalten, bezogen auf das Gesamtgewicht der Zusammensetzungen.

Polymere mit verdickenden Eigenschaften der Komponente c) sind im Rahmen der vorliegenden Erfindung vorzugsweise polymere Materialien mit einem Molekulargewicht oberhalb 5 000 g/mol, die geeignet sind, eine Zusammensetzung enthaltend eine oder mehrere Substanzen ausgewählt aus der Gruppe bestehend aus Wasserstoffperoxid und Wasserstoffperoxid freisetzenden Substanzen bei Einsatzmengen des Polymers von 30 Gew.-% oder weniger, vorzugsweise von 10 Gew.-% oder weniger, besonders bevorzugt von 6 Gew.-% oder weniger und insbesondere bevorzugt von 3 Gew.-% oder weniger, bezogen auf das Gesamtgewicht der Zusammensetzung, signifikant zu verdicken. Diese Verdickung ist vorzugsweise derart, dass die Viskosität der Zusammensetzung in mPa · s bei 20 °C mit den oben genannten Mengen der Polymere um 30 % oder mehr erhöht wird. Bevorzugt sind Polymere, die bei einem pH-Wert von < pH 7, bevorzugt < pH 5, Verdickungsleistung zeigen, da Wasserstoffperoxid unter diesen Bedingungen besonders stabil ist.

Vorzugsweise ist das eine oder sind die mehreren Polymere mit verdickenden Eigenschaften der Komponente c) ausgewählt aus der Gruppe bestehend aus Polymeren mit C-C Backbone (d. h. mit einem Kohlenstoffgrundgerüst, das vorzugsweise durch Polymerisation olefinisch ungesättigter Verbindungen erhalten werden kann), Biopolymeren, modifizierten Biopolymeren, Assoziativverdickern und Polyalkylenglykolen. In einer hierunter bevorzugten Ausführungsform der Erfindung ist das eine oder sind die mehreren Polymere mit verdickenden Eigenschaften der Komponente c) ausgewählt aus der Gruppe bestehend aus Polymeren mit C-C Backbone, Biopolymeren, modifizierten Biopolymeren und Assoziativverdickern.

In einer besonders bevorzugten Ausführungsform der Erfindung ist das eine oder sind die mehreren Polymere mit verdickenden Eigenschaften der Komponente c) ausgewählt aus Polymeren mit C-C-Backbone.

Polymere mit verdickenden Eigenschaften mit C-C Backbone sind insbesondere Polymere auf Basis von Acrylsäure, Methacrylsäure und deren Estern, Acrylamiden und deren Derivaten, Polyvinylalkohol und dessen Derivaten.

Bevorzugt sind hierbei Polyacrylamide, Polyacrylate und modifizierte Polyacrylate, wie sie z. B. unter dem Markennamen Carbomer^{®} erhältlich sind.

Besonders bevorzugt ist jedoch das eine oder sind die mehreren Polymere mit C-C Backbone ausgewählt aus teilweise oder vollständig neutralisierten sulfonsäuregruppenhaltigen Polymeren.

Diese Polymere können vernetzt oder unvernetzt sein. Besonders bevorzugt sind Polymere auf Basis Acrylamidomethylpropansulfonsäure (AMPS^{®}, Lubrizol). Diese Polymere zeigen auch bei pH-Werten von 7 oder kleiner gute Verdickungsleistung und sind deshalb zur Verdickung von Wasserstoffperoxidlösungen besonders geeignet, da Wasserstoffperoxid bei einem pH-Wert von 3-5 maximale Stabilität zeigt.

Insbesondere bevorzugt ist das eine oder sind die mehreren teilweise oder vollständig neutralisierten, sulfonsäuregruppenhaltigen Polymere ausgewählt aus der Gruppe bestehend aus Homo- oder Copolymeren von Acrylamidomethylpropansulfonsäue und deren Salzen.

Unter den soeben genannten Polymeren sind wiederum Polymere mit mindestens 20 mol-% an Einheiten basierend auf Acrylamidomethylpropansulfonsäure und/oder ihren Salzen bevorzugt und Polymere mit mindestens 50 mol-% an Einheiten basierend auf Acrylamidomethylpropansulfonsäure und/oder ihren Salzen besonders bevorzugt, wobei sich die mol-Angaben jeweils auf das gesamte Polymer beziehen.

Im Falle der Copolymere sind neben Struktureinheiten basierend auf Acrylamidomethylpropansulfonsäure und/oder ihren Salzen vorzugsweise ein oder mehrere Struktureinheiten basierend auf den folgenden Comonomeren in den Copolymeren enthalten: Acrylsäure, Methacrylsäure, Acrylamid, Dimethylacrylamid, Vinylpyrrolidon (VP), Hydroxyethylacrylat, Hydroxyethylmethacrylat, Acryl- oder Methacrylsäureester von ethoxylierten Alkoholen RO-(CH₂CH₂O)ₘH, worin R ein Alkylrest mit 12 bis 30 Kohlenstoffatomen und m eine Zahl von 3 bis 30 ist, und CH₂ = CH-COO-(CH₂CH₂-COO)ₙX, worin n eine Zahl von 0 bis 10 ist und X ein Gegenion ist und vorzugsweise H⁺, Na⁺ und/oder NH₄⁺ bedeutet.

Die Polymere ausgewählt aus der Gruppe bestehend aus Homo- oder Copolymeren von Acrylamidomethylpropansulfonsäure und ihren Salzen können vernetzt oder unvernetzt sein. Im Falle der Vernetzung enthalten sie Struktureinheiten basierend auf Monomeren mit 2 oder mehr olefinischen Doppelbindungen. Im Falle der Vernetzung sind derartige Struktureinheiten vorzugsweise von 0,1 bis 10 mol-% in den Homo- oder Copolymeren enthalten, bezogen auf das gesamte Polymer.

Sofern in den Homo- oder Copolymeren von Acrylamidomethylpropansulfonsäure und/oder ihren Salzen ein oder mehrere Struktureinheiten basierend auf Acrylamidomethylpropansulfonsäure und/oder ihren Salzen ein oder mehrere andere Gegenionen als H⁺ aufweisen, sind diese anderen Gegenionen vorzugsweise ausgewählt aus der Gruppe bestehend aus Na⁺ und NH₄⁺.

Geeignete Polymere sind unter anderem in den Offenlegungsschriften EP 0 816 403, EP 1 069 142, EP 1 116 733 und DE 10 2009 014877 (Clariant), EP 1 347 736 (L'Oreal) oder EP 1 496 081 (Seppic) genannt. Beispielhaft seien genannt: Aristoflex^{®} AVC (Ammonium Acryloyldimethyltaurate/VP Copolymer), Aristoflex^{®} AVS (Sodium Acryloyldimethyltaurate/VP Crosspolymer), Aristoflex^{®} TAC (Ammonium Acryloyl Dimethyltaurate Carboxyethyl Acrylate Crosspolymer), Hostacerin^{®} AMP5 (Ammonium Polyacryloyldimethyl Taurate), Aristoflex^{®} HMB (Ammonium Acryloyldimethyltaurate/Beheneth-25 Methacrylate Crosspolymer), Aristoflex^{®} BLV (Ammonium Acryloyldimethyltaurate/Beheneth-25 Methacrylate Crosspolymer), Aristoflex^{®} HMS (Ammonium Acryloyldimethyltaurate/Steareth-25 Methacrylate Crosspolymer), Aristoflex^{®} SNC (Ammonium Acryloyldimethyltaurate/Steareth-8 Methacrylate Copolymer), Aristoflex^{®} LNC (Ammonium Acryloyldimethyltaurate/ Laureth-7 Methacrylate Copolymer) oder Sepinov^{®} EMT10 (Hydroxyethyl Acrylate / Sodium Acryloyldimethyl Taurate Copolymer), Sepigel^{®} 305.

In einer weiteren besonders bevorzugten Ausführungsform der Erfindung ist das eine oder sind die mehreren Polymere mit verdickenden Eigenschaften der Komponente c) ausgewählt aus der Gruppe bestehend aus Biopolymeren und modifizierten Biopolymeren. Hierzu zählen Polymere, deren Grundgerüst aus Zuckermolekülen oder Derivaten von Zuckermolekülen (z. B. Aminozucker, Zuckersäuren, Zuckerester) aufgebaut sind. Zu diesen Polymeren zählen Celluloseether, wie Carboxyethylcellulose oder Hydroxyethylcellulose, Stärke und Stärkederivate, natürliche Gums wie Xanthan Gum, Guar Gum, Hydroxypropylguar, Carrageenan, Pektin, Chitosan und Chitosanderivate. Ebenfalls geeignet sind kationische Biopolymere wie Polyquaternium-10 (quaternierter Celluloseether), kationische Guar Gums (Jaguar^{®}, Rhodia) oder kationische Hydroxypropylguars. Unter den genannten Substanzen sind Xanthan Gum und Celluloseether bevorzugt.

In einer weiteren besonders bevorzugten Ausführungsform der Erfindung ist das eine oder sind die mehreren Polymere mit verdickenden Eigenschaften der Komponente c) ausgewählt aus Assoziatiwerdickern. Assoziativverdicker sind Polymere auf Basis von Polyethylenglykol, die hydrophobe Endgruppen tragen und sowohl mit als auch ohne zusätzliche Tenside Verdickungsleistung zeigen können.
Es handelt sich einerseits um ethoxylierte/propoxylierte Polyole, die mit Fettsäuren endverschlossen sind. Beispiele hierfür sind PEG-150-polyglyceryl-2-tristearate (Genapol^{®} DAT 100, Clariant), PEG-150 Disterate, PEG-150-pentaetrythrityltetrastearate (Crothix^{®}, Croda) und PEG-120 Methylglucose Dioleate (Glucamate^{®} DOE, Lubrizol).
Weiterhin gehören veretherte Polyethylenglykole wie Copolymere aus Ethylenoxid und Dodecandiol sowie Polyurethane auf Basis von Polyethylenglykol zu dieser Gruppe. Beispielhaft seien PEG-150/Stearyl Alcohol/SMDI Copolymer (Aculyn^{®} 46, Rohm & Haas) und PEG-150/Decyl Alcohol/SMDI Copolymer (Aculyn^{®} 44, Rohm & Haas) genannt.
Insbesondere bevorzugt ist der eine oder sind die mehreren Assoziativverdicker jedoch ausgewählt aus Phosphorsäureestern, vorzugsweise aus neutralen Phosphorsäureestern. Beispiele für derartige Phosphorsäureester sind z. B. in den Offenlegungsschriften WO 2009/015856, WO 2009/015857, WO 2009/015858, WO 2009/015859, WO 2009/015860 und WO 2009/095197 genannt. Sie weisen u. a. den Vorteil auf, dass sie bei niedrigem pH-Wert ausreichend hydrolysestabil sind. Ein Beispiel hierfür ist Ceteareth-50 Phosphate (Clariant).

In einer weiteren besonders bevorzugten Ausführungsform der Erfindung ist das eine oder sind die mehreren Polymere mit verdickenden Eigenschaften der Komponente c) ausgewählt aus Polyalkylenglykolen. Diese werden durch Polymerisation von 1,2-Epoxiden gewonnen. Besonders bevorzugt sind hierunter die Polyethylenglykole mit 100 oder mehr und vorzugsweise 100 bis 100 000 Monomereinheiten und Copolymerisate aus Ethylenoxid und Propylenoxid mit 100 oder mehr und vorzugsweise 100 bis 10 000 Monomereinheiten.

Auch Mischungen der oben genannten Polymertypen können in den erfindungsgemäßen Zusammensetzungen verwendet werden.

Das eine oder die mehreren Polymere mit verdickenden Eigenschaften der Komponente c) sind vorzugsweise in Mengen von 0,1 bis 30 Gew.-%, besonders bevorzugt in Mengen von 0,2 bis 10 Gew.%, insbesondere bevorzugt in Mengen von 0,3 bis 6 Gew.-% und außerordentlich bevorzugt in Mengen von 0,3 bis 3 Gew.-% in den erfindungsgemäßen Zusammensetzungen enthalten, bezogen auf das Gesamtgewicht der Zusammensetzungen.

Vorzugsweise ist die eine oder sind die mehreren Substanzen der Komponente d) ausgewählt aus Verbindungen der Formel (I) und deren Salzen 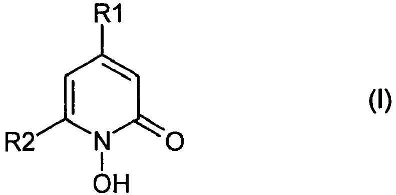 worin R1 H oder ein C₁₋C₄ Alkylrest und R2 H, ein unsubstituierter oder mit Halogen substituierter, verzweigter oder unverzweigter C₁-C₂₀-Alkylrest, ein unsubstituierter oder mit Halogen substituierter C₅₋C₈ Cycloalkylrest, ein unsubstituierter oder mit Halogen substituierter C₆₋C₁₀ Arylrest oder ein unsubstituierter oder mit Halogen substituierter, verzweigter oder unverzweigter C₇-C₂₀ Aralkylrest ist. In einer hierunter bevorzugten Ausführungsform der Erfindung ist in den Verbindungen der Formel (I) R1 ein C₁-C₄ Alkylrest und R2 ein unsubstituierter oder mit Halogen substituierter, verzweigter oder unverzweigter C₁-C₂₀-Alkylrest, ein unsubstituierter oder mit Halogen substituierter C₅-C₈ Cycloalkylrest, ein unsubstituierter oder mit Halogen substituierter C₆-C₁₀ Arylrest oder ein unsubstituierter oder mit Halogen substituierter, verzweigter oder unverzweigter C₇-C₂₀ Aralkylrest.

Vorzugsweise sind die Reste R2 nicht mit Halogen substituiert.

In einer bevorzugten Ausführungsform der Erfindung liegt die eine oder liegen die mehreren Verbindungen der Komponente d) in Form der Säure (Verbindungen der Formel (I)) oder in Form ihrer Alkali-, Erdalkali- oder Aminsalze oder ihrer Salze mit polymeren Gegenionen wie z. B. Polykationpolymeren als Gegenionen in den erfindungsgemäßen Zusammensetzungen vor. In einer hierunter bevorzugten Ausführungsform der Erfindung liegt die eine oder liegen die mehreren Verbindungen der Komponente d) in Form der Säure oder in Form ihrer Alkali-, Erdalkali- oder Aminsalze in den erfindungsgemäßen Zusammensetzungen vor.

In einer besonders bevorzugten Ausführungsform der Erfindung ist R1 in der einen oder in den mehreren Verbindungen der Formel (I) oder in ihren Salzen Methyl und R2 Cyclohexyl oder 2,4,4-Trimethylpentyl.

In einer weiteren besonders bevorzugten Ausführungsform der Erfindung sind R1 und R2 in der einen oder in den mehreren Verbindungen der Formel (I) oder in ihren Salzen H.

Besonders bevorzugt liegen die Verbindungen der Formel (I) in Form ihrer Alkanolaminsalze und insbesondere bevorzugt in Form ihrer Monoethanolaminsalze vor. Beispiele für derartige Salze sind in DE 2234009 erwähnt.

Insbesondere bevorzugt sind dabei 4-Methyl-6-(2,4,4-trimethylpentyl)-1-hydroxy-2-pyridon, das Monoethanolaminsalz von 4-Methyl-6-(2,4,4-trimethylpentyl)-1-hydroxy-2-pyridon (Octopirox^{®}, Clariant) sowie 4-Methyl-6-(cyclohexyl)-1-hydroxy-2-pyridon und das Monoethanolaminsalz von 4-Methyl-6-(cyclohexyl)-1-hydroxy-2-pyridon (Ciclopirox^{®}, Sanofi-Aventis).

Diese Substanzen können anhand literaturbekannter Verfahren erhalten werden, vergleiche hierzu die in DE 2234009 genannten Referenzen.

In den erfindungsgemäßen Zusammensetzungen ist die eine oder sind die mehreren Substanzen der Komponente d) in Mengen von vorzugsweise 0,1 bis 20 000 ppm (0,00001 bis 2 Gew.-%), besonders bevorzugt in Mengen von 0,5 bis 1 000 ppm (0,00005 bis 0,1 Gew.-%) und insbesondere bevorzugt in Mengen von 1 bis 100 ppm (0,0001 bis 0,01 Gew.-%) enthalten, bezogen auf das Gesamtgewicht der Zusammensetzungen.

Die Hydroxypyridone können in den erfindungsgemäßen Zusammensetzungen mit weiteren Stabilisatoren kombiniert werden. Weitere geeignete Stabilisatoren sind z. B. Polyphosphate oder deren Alkali- oder Erdalkalimetallsalze, ,Alkali- oder Erdalkalistannate, Phenacetin und seine Säuresalze sowie Oxychinolin und seine Säuresalze. Im Allgemeinen enthalten die lieferfertigen Wasserstoffperoxidlösungen bereits Stabilisatoren, bevorzugt in Form von Polyphosphaten.

Die erfindungsgemäßen Zusammensetzungen können eingesetzt werden als Bleichzusammensetzungen für Haar oder Zähne, als Bleichkomponente oder Entwickler für oxidative Haarfarben oder als Fixierkomponente für Dauerwellformulierungen und als Haushaltsreiniger. Darüber hinaus können die erfindungsgemäßen Zusammensetzungen als Fleckengele zur Vorbehandlung von Wäsche, Vorwaschsprays, Fleckentferner, Oberflächenreiniger, Toilettenreiniger, Multifunktionsfleckengele oder Bleichgele Verwendung finden. Eine weitere mögliche Verwendung sind Haarfärbezusammensetzungen zur Verhinderung von Bleichschäden bei der Haarfärbung.

Die Viskosität der erfindungsgemäßen Zusammensetzungen beträgt vorzugsweise 50 - 100 000 mPa · s bei 20 °C, besonders bevorzugt 100 - 20 000 mPa · s bei 20 °C und insbesondere bevorzugt 150 - 5 000 mPa · s bei 20 °C. Die Viskositäten werden an den erfindungsgemäßen Zusammensetzungen selbst mit einem Brookfield-Viskosimeter Model DV II, bei 20 Umdrehungen / Minute und 20 °C gemessen. Es werden die Spindeln 1 bis 7 aus dem Spindelset RV verwendet. Unter diesen Messbedingungen wird Spindel 1 für Viskositäten von maximal 500 mPa · s, Spindel 2 für Viskositäten von maximal 1 000 mPa · s, Spindel 3 für Viskositäten von maximal 5 000 mPa · s, Spindel 4 für Viskositäten von maximal 10 000 mPa · s, Spindel 5 für Viskositäten von maximal 20 000 mPa · s, Spindel 6 für Viskositäten von maximal 50 000 mPa · s und Spindel 7 für Viskositäten von maximal 200 000 mPa · s gewählt.

In einer bevorzugten Ausführungsform der Erfindung sind die erfindungsgemäßen Zusammensetzungen ölfreie Zusammensetzungen mit gelförmiger Konsistenz.

In einer weiteren bevorzugten Ausführungsform der Erfindung sind die erfindungsgemäßen Zusammensetzungen Emulsionen. Bei den Emulsionen handelt es sich bevorzugt um Öl-in-Wasser-Emulsionen oder Mikroemulsionen.

Der nichtwässrige Anteil dieser Emulsionen, der sich weitgehend aus dem Emulgator und dem Ölkörper zusammensetzt, liegt üblicherweise bei 0,5 bis 20,0 Gew.-% und vorzugsweise bei 1,0 bis 10,0 Gew.-%, bezogen auf das Gesamtgewicht der Emulsionen. Daraus folgt, dass die Emulsionen 80,0 bis 99,5 Gew.-% und vorzugsweise 90,0 bis 99,0 Gew.-% der wässrigen Phase enthalten können, bezogen auf das Gesamtgewicht der Emulsionen.

Die erfindungsgemäßen Zusammensetzungen können auch anionische, kationische, nichtionische, ampholytische Tenside und/oder Betaintenside enthalten.

Die Gesamtmenge der in den erfindungsgemäßen Zusammensetzungen eingesetzten Tenside beträgt, bezogen auf das Gesamtgewicht der Zusammensetzungen, vorzugsweise von 0,1 bis 20 Gew.-%, besonders bevorzugt von 0,5 bis 10,0 Gew.-% und insbesondere bevorzugt von 1,0 bis 5,0 Gew.-%.

Als anionische Tenside bevorzugt sind (C₁₀-C₂₂)-Alkyl- und Alkylen-carboxylate, Alkylethercarboxylate, Fettalkoholsulfate, Fettalkoholethersulfate, Alkylamidsulfate und -sulfonate, Fettsäurealkylamidpolyglykolethersulfate, Alkansulfonate und Hydroxyalkansulfonate, Olefinsulfonate, Acylester von Isethionaten, α-Sulfofettsäureester, Alkylbenzolsulfonate, Alkylphenolglykolethersulfonate, Sulfosuccinate, Sulfobernsteinsäurehalbester und -diester, Fettalkoholphosphate, Fettalkoholetherphosphate, Eiweiß-Fettsäure-Kondensationsprodukte, Alkylmonoglyceridsulfate und -sulfonate, Alkylglyceridethersulfonate, Fettsäuremethyltauride, Fettsäuresarkosinate, Sulforicinoleate, Acylglutamate und Acylglycinate. Diese Verbindungen und deren Mischungen werden in Form ihrer wasserlöslichen oder in Wasser dispergierbaren Salze benutzt, beispielsweise der Natrium-, Kalium-, Magnesium-, Ammonium-, Mono-, Di- und Triethanolammonium- sowie analogen Alkylammonium-Salze.

Die Menge der anionischen Tenside in den erfindungsgemäßen Zusammensetzungen beträgt bevorzugt von 0,05 bis 20,0 Gew.-%, besonders bevorzugt von 0,5 bis 10,0 Gew.-% und insbesondere bevorzugt von 1,0 bis 5,0 Gew.-%, bezogen auf das Gesamtgewicht der Zusammensetzungen.

Bevorzugte kationische Tenside sind quartäre Ammonium-Salze, wie Di-(C₈-C₂₂)-Alkyl-dimethylammoniumchlorid oder -bromid, vorzugsweise Di-(C₈-C₂₂)-Alkyldimethylammoniumchlorid oder -bromid; (C₈-C₂₂)-Alkyl-dimethylethylammoniumchlorid oder -bromid; (C₈-C₂₂)-Alkyl-trimethylammoniumchlorid oder -bromid, vorzugsweise Cetyltrimethylammoniumchlorid oder -bromid und (C₈-C₂₂)-Alkyl-trimethylammoniumchlorid oder -bromid; (C₁₀-C₂₄)-Alkyldimethylbenzyl-ammoniumchlorid oder -bromid, vorzugsweise (C₁₂-C₁₈)-Alkyldimethylbenzyl-ammoniumchlorid, (C₈-C₂₂)-Alkyl-dimethylhydroxyethylammoniumchlorid, -phosphat, -sulfat, -lactat, (C₈-C₂₂)-Alkylamidopropyltrimethylammoniumchlorid, -methosulfat, N,N-bis(2-C₈-C₂₂-Alkanoyl-oxyethyl)-dimethylammoniumchlorid, -methosulfat, N,N-bis(2-C₈-C₂₂-Alkanoyl-oxyethyl)hydroxyethyl-methyl-ammoniumchlorid, -methosulfat, sowie Esterquats auf Basis von C₈-C₂₂ Alkanoylestern von Triethanolamin oder Methyldiethanolamin.

Die Menge der kationischen Tenside in den erfindungsgemäßen Zusammensetzungen beträgt bevorzugt von 0,1 bis 10,0 Gew.-%, besonders bevorzugt von 0,5 bis 7,0 Gew.-% und insbesondere bevorzugt von 1,0 bis 5,0 Gew.-%, bezogen auf das Gesamtgewicht der Zusammensetzungen.

Als nichtionische Tenside bevorzugt sind Fettalkoholethoxylate (Alkylpolyethylenglykole); Alkylphenolpolyethylenglykole; Fettaminethoxylate (Alkylaminopolyethylenglykole); Fettsäureethoxylate (Acylpolyethylenglykole); Polypropylenglykolethoxylate (Pluronics^{®}); Fettsäurealkanolamide, (Fettsäureamidpolyethylenglykole); Saccharoseester; Sorbitester und Sorbitanester und deren Polyglykolether, sowie C₈-C₂₂-Alkylpolyglucoside.

Die Menge der nichtionischen Tenside in den erfindungsgemäßen Zusammensetzungen (z. B. im Falle von Rinse-off-Produkten) liegt bevorzugt im Bereich von 0,05 bis 20,0 Gew.-%, besonders bevorzugt von 0,1 bis 10,0 Gew.-% und insbesondere bevorzugt von 0,5 bis 5,0 Gew.-%, bezogen auf das Gesamtgewicht der Zusammensetzungen.

Weiterhin können die erfindungsgemäßen Zusammensetzungen amphotere Tenside enthalten. Diese können beschrieben werden als Derivate langkettiger sekundärer oder tertiärer Amine, die über eine Alkylgruppe mit 8 bis 18 C-Atomen verfügen und bei denen eine weitere Gruppe substituiert ist mit einer anionischen Gruppe, die die Wasserlöslichkeit vermittelt, so z. B. mit einer Carboxyl-, Sulfat- oder Sulfonat-Gruppe. Bevorzugte Amphotenside sind N-(C₁₂-C₁₈)-Alkyl-β-aminopropionate und N-(C₁₂-C₁₈)-Alkyl-β-iminodipropionate als Alkali- und Mono-, Di- und Trialkylammonium-Salze. Geeignete weitere Tenside sind auch Aminoxide. Es sind dies Oxide tertiärer Amine mit einer langkettigen Gruppe von 8 bis 18 C-Atomen und zwei meist kurzkettigen Alkylgruppen mit 1 bis 4 C-Atomen. Bevorzugt sind hier beispielsweise die C₁₀- bis C₁₈-Alkyldimethylaminoxide, Fettsäureamidoalkyl-dimethylaminoxid.

Eine weitere bevorzugte Gruppe von Tensiden sind Betaintenside, auch zwitterionische Tenside genannt. Diese enthalten im selben Molekül eine kationische Gruppe, insbesondere eine Ammonium-Gruppe und eine anionische Gruppe, die eine Carboxylat-Gruppe, Sulfat-Gruppe oder Sulfonat-Gruppe sein kann. Geeignete Betaine sind vorzugsweise Alkylbetaine wie Coco-Betain oder Fettsäurealkylamidopropylbetaine, beispielsweise Kokosacylamidopropyldimethylbetain oder die C₁₂- bis C₁₈-Dimethylaminohexanoate bzw. die C₁₀- bis C₁₈-Acylamidopropandimethylbetaine.

Die Menge der amphoteren Tenside und/oder Betaintenside in den erfindungsgemäßen Zusammensetzungen beträgt bevorzugt von 0,5 bis 20,0 Gew.-% und besonders bevorzugt von 1,0 bis 10,0 Gew.-%, bezogen auf das Gesamtgewicht der Zusammensetzungen.

Bevorzugte Tenside sind Laurylsulfat, Laurethsulfat, Cocoamidopropylbetain, Alkylbetaine wie Coco-Betain, Natriumcocoylglutamat und Lauroamphoacetat.

Die erfindungsgemäßen Zusammensetzungen können als weitere Hilfs- und Zusatzstoffe Ölkörper, Silikonöle, Wachse, Emulgatoren, Co-Emulgatoren, Solubilisatoren, kationische Polymere, Filmbildner, Überfettungsmittel, Rückfetter, antimikrobielle Wirkstoffe, Feuchthaltemittel, Lösemittel, Farbstoffe, Duftstoffe, Perlglanzmittel und/oder Trübungsmittel enthalten.

Die Ölkörper können vorteilhafterweise ausgewählt werden aus den Gruppen der Triglyceride, natürlichen und synthetischen Fettkörper, vorzugsweise Ester von Fettsäuren mit Alkoholen niedriger C-Zahl, z. B. mit Isopropanol, Propylenglykol oder Glycerin, oder Ester von Fettalkoholen mit Alkansäuren niedriger C-Zahl oder mit Fettsäuren oder aus der Gruppe der Alkylbenzoate, sowie natürlichen oder synthetischen Kohlenwasserstoffölen.

In Betracht kommen Triglyceride von linearen oder verzweigten, gesättigten oder ungesättigten, gegebenenfalls hydroxylierten, C₈-C₃₀-Fettsäuren, insbesondere pflanzliche Öle, wie Sonnenblumen-, Mais-, Soja-, Reis-, Jojoba-, Babusscu-, Kürbis-, Traubenkern-, Sesam-, Walnuss-, Aprikosen-, Orangen-, Weizenkeim-, Pfirsichkern-, Makadamia-, Avocado-, Süßmandel-, Wiesenschaumkraut-, Ricinusöl, Olivenöl, Erdnussöl, Rapsöl und Kokosnussöl, sowie synthetische Triglyceridöle, z. B. das Handelsprodukt Myritol^{®} 318. Auch gehärtete Triglyceride sind erfindungsgemäß bevorzugt. Auch Öle tierischen Ursprungs, beispielsweise Rindertalg, Perhydrosqualen, Lanolin können eingesetzt werden.

Eine weitere Klasse von bevorzugten Ölkörpern sind die Benzoesäureester von linearen oder verzweigten C₈-₂₂-Alkanolen, z. B. die Handelsprodukte Finsolv^{®} SB (Isostearylbenzoat), Finsolv^{®} TN (C₁₂-C₁₅-Alkylbenzoat) und Finsolv^{®} EB (Ethylhexylbenzoat).

Eine weitere Klasse von bevorzugten Ölkörpern sind die Dialkylether mit insgesamt 12 bis 36 Kohlenstoffatomen, insbesondere mit 12 bis 24 Kohlenstoffatomen, wie z. B. Di-n-octylether (Cetiol^{®} OE), Di-n-nonylether, Di-n-decylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether, Di-3-ethyldecylether, tert.-Butyl-n-octylether, iso-Pentyl-n-octylether und 2-Methyl-pentyl-n-octylether sowie Di-tert.-butylether und Di-iso-pentylether.

Ebenso in Betracht kommen verzweigte gesättigte oder ungesättigte Fettalkohole mit 6 - 30 Kohlenstoffatomen, z. B. Isostearylalkohol, sowie Guerbetalkohole.

Eine weitere Klasse von bevorzugten Ölkörpern sind Hydroxycarbonsäurealkylester. Bevorzugte Hydroxycarbonsäurealkylester sind Vollester der Glykolsäure, Milchsäure, Apfelsäure, Weinsäure oder Zitronensäure. Weitere grundsätzlich geeignete Ester der Hydroxycarbonsäuren sind Ester der β-Hydroxypropionsäure, der Tartronsäure, der D-Gluconsäure, Zuckersäure, Schleimsäure oder Glucuronsäure. Als Alkoholkomponente dieser Ester eignen sich primäre, lineare oder verzweigte aliphatische Alkohole mit 8 bis 22 C-Atomen. Dabei sind die Ester von C₁₂-C₁₅-Fettalkoholen besonders bevorzugt. Ester dieses Typs sind im Handel erhältlich, z. B. unter dem Handelsnamen Cosmacol^{®} der EniChem, Augusta Industriale.

Eine weitere Klasse von bevorzugten (Olkörpern sind Dicarbonsäureester von linearen oder verzweigten C₂-C₁₀-Alkanolen, wie Di-n-butyladipat (Cetiol^{®} B), Di-(2-ethylhexyl)-adipat und Di-(2-ethylhexyl)-succinat sowie Diolester wie Ethylenglycol-dioleat, Ethylenglycol-di-isotridecanoat, Propylenglycol-di-(2-ethylhexanoat), Propylenglycol-di-isostearat, Propylenglycol-di-pelargonat, Butandiol-di-isostearat und Neopentylglycoldicaprylat sowie Di-isotridecylacelaat.

Ebenso bevorzugte Ölkörper sind symmetrische, unsymmetrische oder cyclische Ester der Kohlensäure mit Fettalkoholen, Glycerincarbonat oder Dicaprylylcarbonat (Cetiol^{®} CC).

Eine weitere Klasse von bevorzugten Ölkörpern sind die Ester von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertig linearen oder verzweigten C₂-C₆-Alkanolen.

Eine weitere Klasse von bevorzugten Ölkörpern sind Kohlenwasserstofföle, zum Beispiel solche mit linearen oder verzweigten, gesättigten oder ungesättigten C₇-C₄₀-Kohlenstoffketten, beispielsweise Vaseline, Dodecan, Isododecan, Cholesterol, Lanolin, synthetische Kohlenwasserstoffe wie Polyolefine, insbesondere Polyisobuten, hydriertes Polyisobuten, Polydecan, sowie Hexadecan, Isohexadecan, Paraffinöle, Isoparaffinöle, z. B. die Handelsprodukte der Permethyl^{®}-Serie, Squalan, Squalen, und alicyclische Kohlenwasserstoffe, z. B. das Handelsprodukt 1,3-Di-(2-ethyl-hexyl)-cyclohexan (Cetiol^{®} S), Ozokerit, und Ceresin.

An Silikonölen bzw. -wachsen stehen vorzugsweise zur Verfügung Dimethylpolysiloxane und Cyclomethicone, Polydialkylsiloxane R₃SiO(R₂SiO)ₓSiR₃, wobei R für Methyl oder Ethyl, besonders bevorzugt für Methyl, steht und x für eine Zahl von 2 bis 500 steht, beispielsweise die unter den Handelsnamen VICASIL (General Electric Company), DOW CORNING 200, DOW CORNING 225, DOW CORNING 200 (Dow Corning Corporation), erhältlichen Dimethicone, sowie die unter SilCare^{®} Silicone 41 M65, SilCare^{®} Silicone 41M70, SilCare^{®} Silicone 41M80 (Clariant) erhältlichen Dimethicone, Stearyldimethylpolysiloxan, C₂₀-C₂₄-Alkyl-dimethylpolysiloxan, C₂₄-C₂₈-Alkyldimethylpolysiloxan, aber auch die unter SilCare^{®} Silicone 41M40, SilCare^{®} Silicone 41M50 (Clariant) erhältlichen Methicone, weiterhin Trimethylsiloxysilicate [(CH₂)₃SiO)_{1/2}]ₓ[SiO₂]_{y}, wobei x für eine Zahl von 1 bis 500 und y für eine Zahl von 1 bis 500 steht, Dimethiconole R₃SiO[R₂SiO]ₓSiR₂OH und HOR₂SiO[R₂SiO]ₓSiR₂OH, wobei R für Methyl oder Ethyl und x für eine Zahl bis zu 500 steht, Polyalkylarylsiloxane, beispielsweise die unter den Handelsbezeichnungen SF 1075 METHYLPHENYL FLUID (General Electric Company) und 556 COSMETIC GRADE PHENYL TRIMETHICONE FLUID (Dow Corning Corporation) erhältlichen Polymethylphenylsiloxane, Polydiarylsiloxane, Silikonharze, cyclische Silikone und amino-, fettsäure-, alkohol-, polyether-, epoxy-, fluor- und/oder alkylmodifizierte Silikonverbindungen, sowie Polyethersiloxan-Copolymere.

Die erfindungsgemäßen Zusammensetzungen können Wachse, beispielsweise Paraffinwachse, Mikrowachse und Ozokerite, Bienenwachs und ihre Teilfraktionen sowie der Bienenwachsderivate, Wachse aus der Gruppe der homopolymeren Polyethylene oder Copolymere der α-Olefine, sowie natürliche Wachse wie Reiswachs, Candellilawachs, Carnaubawachs, Japanwachs oder Schellackwachs enthalten.

Als Emulgatoren, Co-Emulgatoren und Solubilisatoren können nichtionische, anionische, kationische oder amphotere oberflächenaktive Verbindungen eingesetzt werden.

Als nichtionogene oberflächenaktive Verbindungen kommen vorzugsweise in Betracht:
Anlagerungsprodukte von 0 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe und an Sorbitan- bzw. Sorbitolester; (C₁₂-C₁₈)-Fettsäuremono- und -diester von Anlagerungsprodukten von 0 bis 30 Mol Ethylenoxid an Glycerin; Glycerinmono- und -diester und Sorbitanmono- und -diester von gesättigten und ungesättigten Fettsäuren mit 6 bis 22 Kohlenstoffatomen und gegebenenfalls deren Ethylenoxidanlagerungsprodukten; Anlagerungsprodukte von 15 bis 60 Mol Ethylenoxid an Rizinusöl und/oder gehärtetes Rizinusöl; Polyol- und insbesondere Polyglycerinester, wie z. B. Polyglycerinpolyricinoleat und Polyglycerinpoly-12-hydroxystearat. Ebenfalls vorzugsweise geeignet sind ethoxylierte Fettamine, Fettsäureamide, Fettsäurealkanolamide und Gemische von Verbindungen aus mehreren dieser Substanzklassen.

Als ionogene Co-Emulgatoren eignen sich z. B. anionische Emulgatoren, wie mono-, di- oder tri-Phosphorsäureester, Seifen (z. B. Natriumstearat), Fettalkoholsulfate aber auch kationische Emulgatoren wie mono-, di- und tri-Alkylquats und deren polymere Derivate.

An amphoteren Emulgatoren stehen vorzugsweise zur Verfügung Alkylaminoalkylcarbonsäuren, Betaine, Sulfobetaine und Imidazolinderivate.

Besonders bevorzugt zum Einsatz kommen Fettalkoholethoxylate, gewählt aus der Gruppe der ethoxylierten Stearylalkohole, Isostearylalkohole, Cetylalkohole, Isocetylalkohole, Oleylalkohole, Laurylalkohole, Isolaurylalkohole, Cetylstearylalkohole, insbesondere Polyethylenglycol(13)stearylether, Polyethylenglycol(14)stearylether, Polyethylenglycol(15)stearylether, Polyethylenglycol(16)stearylether, Polyethylenglycol(17)stearylether, Polyethylenglycol(18)stearylether, Polyethylenglycol(19)stearylether, Polyethylenglycol(20)stearylether, Polyethylenglycol(12)isostearylether, Polyethylenglycol(13)isostearylether, Polyethylenglycol(14)isostearylether, Polyethylenglycol(15)isostearylether, Polyethylenglycol(16)isostearylether, Polyethylenglycol(17)isostearylether, Polyethylenglycol(18)isostearylether, Polyethylenglycol(19)isostearylether, Polyethylenglycol(20)isostearylether, Polyethylenglycol(13)cetylether, Polyethylenglycol(14)cetylether, Polyethylenglycol(15)cetylether, Polyethylenglycol(16)cetylether, Polyethylenglycol(17)cetylether, Polyethylenglycol(18)cetylether, Polyethylenglycol(19)cetylether, Polyethylenglycol(20)cetylether, Polyethylenglycol(13)isocetylether, Polyethylenglycol(14)isocetylether, Polyethylenglycol(15)isocetylether, Polyethylenglycol(16)isocetylether, Polyethylenglycol(17)isocetylether, Polyethylenglycol(18)isocetylether, Polyethylenglycol(19)isocetylether, Polyethylenglycol(20)isocetylether, Polyethylenglycol(12)oleylether, Polyethylenglycol(13)oleylether, Polyethylenglycol(14)oleylether, Polyethylenglycol(15)oleylether, Polyethylenglycol(12)laurylether, Polyethylenglycol(12)isolaurylether, Polyethylenglycol(13)cetylstearylether, Polyethylenglycol(14)cetylstearylether, Polyethylenglycol(15)cetylstearylether, Polyethylenglycol(16)cetylstearylether, Polyethylenglycol(17)cetylstearylether, Polyethylenglycol(18)cetylstearylether, Polyethylenglycol(19)cetylstearylether.

Ebenso bevorzugt sind Fettsäureethoxylate, gewählt aus der Gruppe der ethoxylierten Stearate, Isostearate und Oleate, insbesondere Polyethylenglycol(20)stearat, Polyethylenglykol(21)stearat, Polyethylenglykol(22)stearat, Polyethylenglykol(23)stearat, Polyethylenglykol(24)stearat, Polyethylenglykol(25)stearat, Polyethylenglykol(12)isostearat, Polyethylenglykol(13)isostearat, Polyethylenglykol(14)isostearat, Polyethylenglykol(15)isostearat, Polyethylenglykol(16)isostearat, Polyethylenglykol(17)isostearat, Polyethylenglykol(18)isostearat, Polyethylenglykol(19)isostearat, Polyethylenglykol(20)isostearat, Polyethylenglykol(21)isostearat, Polyethylenglykol(22)isostearat, Polyethylenglykol(23)isostearat, Polyethylenglykol(24)isostearat, Polyethylenglykol(25)isostearat, Polyethylenglykol(12)oleat, Polyethylenglykol(13)oleat, Polyethylenglykol(14)oleat, Polyethylenglykol(15)oleat, Polyethylenglykol(16)oleat, Polyethylenglykol(17)oleat, Polyethylenglykol(18)oleat, Polyethylenglykol(19)oleat, Polyethylenglykol(20)oleat.

Als ethoxylierte Alkylethercarbonsäure oder deren Salze kann vorteilhafterweise das Natrium Laureth-11-carboxylat verwendet werden.

Weiterhin ist es von Vorteil, die Polyethylenglykolglycerinfettsäureester aus der Gruppe Polyethylenglykol(20)glyceryllaurat,
Polyethylenglykol(6)glycerylcaprat/caprinat, Polyethylenglykol(20)glyceryloleat,
Polyethylenglykol(20)glycerylisostearat und
Polyethylenglykol(18)glyceryloleat/cocoat zu wählen.

Unter den Sorbitanestern eignen sich besonders
Polyethylenglykol(20)sorbitanmonolaurat,
Polyethylenglycol(20)sorbitanmonostearat,
Polyethylenglycol(20)sorbitanmonoisostearat,
Polyethylenglycol(20)sorbitanmonopalmitat,
Polyethylenglycol(20)sorbitanmonooleat.

Besonders vorteilhafte Coemulgatoren sind Glycerylmonostearat, Glycerylmonooleat, Diglycerylmonostearat, Glycerylisostearat, Polyglyceryl-3-oleat, Polyglyceryl-3-diisostearat, Polyglyceryl-4-isostearat, Polyglyceryl-2-dipolyhydroxystearat, Polyglyceryl-4-dipolyhydroxystearat, PEG-30-dipolyhydroxystearat, Diisostearoylpolyglyceryl-3-diisostearat, Glycoldistearat und Polyglyceryl-3-dipolyhydroxystearat, Sorbitanmonoisostearat, Sorbitanstearat, Sorbitanoleat, Saccharosedistearat, Lecithin, PEG-7-hydriertes Ricinusöl, Cetylalkohol, Stearylalkohol, Behenylalkohol, Isobehenylalkohol und Polyethylenglycol(2)stearylether (Steareth-2), Alkylmethiconcopolyole und Alkyl-Dimethiconcopolyole, insbesondere Cetyldimethiconcopolyol, Laurylmethiconcopolyol.

Die erfindungsgemäßen Zusammensetzungen können einen oder mehrere der Emulgatoren, Co-Emulgatoren oder Solubilisatoren in Mengen von 0,1 bis 20,0 Gew.-%, vorzugsweise von 1,0 bis 15,0 Gew.-% und besonders bevorzugt von 3,0 bis 10,0 Gew.-%, bezogen auf das Gesamtgewicht der Zusammensetzungen, enthalten.

Als kationische Polymere eignen sich die unter der INCI-Bezeichnung "Polyquaternium" bekannten, insbesondere Polyquaternium-31, Polyquaternium-16, Polyquaternium-24, Polyquaternium-7, Polyquaternium-22, Polyquaternium-39, Polyquaternium-28, Polyquaternium-2, Polyquaternium-10, Polyquaternium-11, sowie Polyquaternium 37&mineral oil&PPG trideceth (Salcare SC95), PVP-dimethylaminoethylmethacrylat-Copolymer, Guarhydroxypropyltriammonium-chloride, sowie Calciumalginat und Ammoniumalginat. Des Weiteren können eingesetzt werden kationische Cellulosederivate; kationische Stärke; Copolymere von Diallylammoniumsalzen und Acrylamiden; quaternierte Vinylpyrrolidon/Vinylimidazol-Polymere; Kondensationsprodukte von Polyglykolen und Aminen; quaternierte Kollagenpolypeptide; quaternierte Weizenpolypeptide; Polyethylenimine; kationische Siliconpolymere, wie z. B. Amidomethicone; Copolymere der Adipinsäure und Dimethylaminohydroxypropyldiethylentriamin; Polyaminopolyamid und kationische Chitinderivate, wie beispielsweise Chitosan.

Die erfindungsgemäßen Zusammensetzungen können einen oder mehrere der oben genannten kationischen Polymere in Mengen von 0,1 bis 5,0 Gew.-%, vorzugsweise von 0,2 bis 3,0 Gew.-% und besonders bevorzugt von 0,5 bis 2,0 Gew.-%, bezogen auf das Gesamtgewicht der Zusammensetzungen, enthalten.

Des Weiteren können die erfindungsgemäßen Zusammensetzungen Filmbildner enthalten, die je nach Anwendungszweck z. B. ausgewählt sind aus Salzen der Phenylbenzimidazolsulfonsäure, wasserlöslichen Polyurethanen, beispielsweise C₁₀-Polycarbamylpolyglycerylester, Polyvinylalkohol, Polyvinylpyrrolidoncopolymeren, beispielsweise Vinylpyrrolidon/Vinylacetatcopolymer, wasserlöslichen Acrylsäurepolymeren/Copolymeren bzw. deren Estern oder Salzen.

Die erfindungsgemäßen Zusammensetzungen können einen oder mehrere Filmbildner in Mengen von 0,1 bis 10,0 Gew.-%, vorzugsweise von 0,2 bis 5,0 Gew.-% und besonders bevorzugt von 0,5 bis 3,0 Gew.-%, bezogen auf das Gesamtgewicht der Zusammensetzungen, enthalten.

Als Überfettungsmittel können vorzugsweise Lanolin und Lecithin, nicht ethoxylierte und polyethoxylierte oder acylierte Lanolin- und Lecithinderivate, Polyolfettsäureester, Mono-, Di- und Triglyceride und/oder Fettsäurealkanolamide, wobei die letzteren gleichzeitig als Schaumstabilisatoren dienen, verwendet werden, die bevorzugt in Mengen von 0,01 bis 10,0 Gew.-%, besonders bevorzugt von 0,1 bis 5,0 Gew.-% und insbesondere bevorzugt von 0,5 bis 3,0 Gew.-% eingesetzt werden, bezogen auf das Gesamtgewicht der Zusammensetzungen.

An antimikrobiellen Wirkstoffen können Cetyltrimethylammoniumchlorid, Cetylpyridiniumchlorid, Benzethoniumchlorid, Diisobutylethoxyethyldimethylbenzylammoniumchlorid, Natriumaluminiumchlorohydroxylactat, Triethylcitrat, 2,4,4'-Trichloro-2'-hydroxydiphenylether (Triclosan), 3,4,4'-Trichlorocarbanilid (Triclocarban), Diaminoalkylamid, beispielsweise L-Lysinhexadecylamid, Citratschwermetallsalze, Salicylate, Pyrithione und deren Schwermetallsalze, insbesondere Zinkpyrithion, Farnesol, Ketoconazol, Oxiconazol, Bifonazole, Butoconazole, Cloconazole, Clotrimazole, Econazole, Enilconazole, Fenticonazole, Isoconazole, Miconazole, Sulconazole, Tioconazole Fluconazole, Itraconazole, Terconazole, Naftifine und Terbinafine, Selendisulfid, lodopropynylbutylcarbamat, Methylchloroisothiazolinon, Methylisothiazolinon, Methyldibromo Glutaronitril, AgCl, Chloroxylenol, Natriumbenzoat, sowie Phenoxyethanol, Phenoxyisopropanol, Parabene, bevorzugt Butyl-, Ethyl-, Methyl- und Propylparaben, sowie deren Na-Salze, Pentandiol, 1,2-Hexandiol, 1,2-Octandiol, 2-Bromo-2-Nitropropan-1,3-diol, Ethylhexylglycerin, Benzylalkohol, Sorbinsäure, Benzoesäure, Milchsäure, Imidazolidinylharnstoff, Diazolidinylharnstoff, Dimethyloldimethylhydantoin (DMDMH), Na-Salz von Hydroxymethylglycinat eingesetzt werden.

Die erfindungsgemäßen Zusammensetzungen enthalten die antimikrobiellen Wirkstoffe bevorzugt in Mengen von 0,001 bis 5,0 Gew.-%, besonders bevorzugt von 0,01 bis 3,0 Gew.-% und insbesondere bevorzugt von 0,1 bis 2,0 Gew.-%, bezogen auf das Gesamtgewicht der Zusammensetzungen.

Des Weiteren können Feuchthaltemittel, ausgewählt aus dem Natriumsalz von 2-Pyrrolidone-5-carboxylat (NaPCA), Guanidin; Glycolsäure und deren Salzen, Milchsäure und deren Salzen, Glucosamine und deren Salzen, Lactamidmonoethanolamin, Acetamidmonoethanolamin, Harnstoff, Hydroxysäuren, Panthenol und dessen Derivaten, beispielsweise D-Panthenol (R-2,4-Dihydroxy-N-(3-hydroxypropyl)-3,3-dimethylbutamid), DL-Panthenol, Calciumpantothenat, Panthetin, Pantothein, Panthenylethylether, Isopropylpalmitat, Glycerin und/oder Sorbitol eingesetzt werden, bevorzugt in Mengen von 0,1 bis 15,0 Gew.% und besonders bevorzugt von 0,5 bis 5,0 Gew.-%, bezogen auf das Gesamtgewicht der Zusammensetzungen.

Zusätzlich können die erfindungsgemäßen Zusammensetzungen organische Lösungsmittel enthalten. Prinzipiell kommen als organische Lösungsmittel alle ein- oder mehrwertigen Alkohole in Betracht. Bevorzugt werden Alkohole mit 1 bis 4 Kohlenstoffatomen wie Ethanol, Propanol, Isopropanol, n-Butanol, i-Butanol, t-Butanol, Glycerin und Mischungen aus den genannten Alkoholen eingesetzt. Weitere bevorzugte Alkohole sind Polyethylenglykole mit einer relativen Molekülmasse unter 2 000. Insbesondere ist ein Einsatz von Polyethylenglykol mit einer relativen Molekülmasse zwischen 200 und 600 und in Mengen bis zu 45,0 Gew.-% und von Polyethylenglykol mit einer relativen Molekülmasse zwischen 400 und 600 in Mengen von 5,0 bis 25,0 Gew.-% bevorzugt. Weitere geeignete Lösungsmittel sind beispielsweise Triacetin (Glycerintriacetat) und 1-Methoxy-2-propanol.

Die erfindungsgemäßen Zusammensetzungen können Farbstoffe- und -pigmente, sowohl organische als auch anorganische Farbstoffe, enthalten, die aus der entsprechenden Positivliste der Kosmetikverordnung bzw. der EG-Liste kosmetischer Färbemittel ausgewählt sind.

| Chemische oder sonstige Bezeichnung | CIN | Farbe |
|---|---|---|
| Pigment Green | 10006 | grün |
| Acid Green 1 | 10020 | grün |
| 2,4-Dinitrohydroxynaphtalin-7-sulfosäure | 10316 | gelb |
| Pigment Yellow 1 | 11680 | gelb |
| Pigment Yellow 3 | 11710 | gelb |
| Pigment Orange 1 | 11725 | orange |
| 2,4-Dihydroxyazobenzol | 11920 | orange |
| Solvent Red 3 | 12010 | rot |
| 1-(2'-Chlor-4'-nitro-1'-phenylazo)-2-hydroxynaphthalin | 12085 | rot |
| Pigment Red 3 | 12120 | rot |
| Ceresrot; Sudanrot; Fettrot G | 12150 | rot |
| Pigment Red 112 | 12370 | rot |
| Pigment Red 7 | 12420 | rot |
| Pigment Brown 1 | 12480 | braun |
| 4-(2'-Methoxy-5'-sulfosäurediethylamid-1'-phenylazo)-3-hydroxy-5"-chloro-2",4"-dimethoxy-2-naphthoesäureanilid | 12490 | rot |
| Disperse Yellow 16 | 12700 | gelb |
| 1-(4-Sulfo-1-phenylazo)-4-amino-brezol-sulfosäure | 13015 | gelb |
| 2,4-Dihydroxy-azobenzol-4'-sulfosäure | 14270 | orange |
| 2-(2,4-Dimethylphenylazo-5-sulfosäurse)-1-hydroxynaphthalin-4-sulfosäure | 14700 | rot |
| 2-(4-Sulfo-1-naphthylazo)-1-naphthol-4-sulfosäure | 14720 | rot |
| 2-(6-Sulfo-2,4-xylylazo)-1-naphthol-5-sulfosäure | 14815 | rot |
| 1-(4'-Sulfophenylazo)-2-hydroxynaphthalin | 15510 | orange |
| 1-(2-Sulfosäure-4-chlor-5-carbonsäure-1-phenylazo)-2-hydroxynaphthalin | 15525 | rot |
| 1-(3-Methyl-phenylazo-4-sulfosäure)-2-hydroxynaphthalin | 15580 | rot |
| 1-(4',(8')-Sulfosäurenaphthylazo)-2-hydroxynaphthalin | 15620 | rot |
| 2-Hydroxy-1,2'-azonaphthalin-1'-sulfosäure | 15630 | rot |
| 3-Hydroxy-4-phenylazo-2-naphthylcarbonsäure | 15800 | rot |
| 1-(2-Sulfo-4-methyl-1-phenylazo)-2-naphthylcarbonsäure | 15850 | rot |
| 1-(2-Sulfo-4-methyl-5-chlor-1-phenylazo)-2-hydroxynaphthalin-3-carbonsäure | 15865 | rot |
| 1-(2-Sulfo-1-naphthylazo)-2-hydroxynaphthalin-3-carbonsäure | 15880 | rot |
| 1-(3-Sulfo-1-phenylazo)-2-naphthol-6-sulfosäure | 15980 | orange |
| 1-(4-Sulfo-1-phenylazo)-2-naphthol-6-sulfosäure | 15985 | gelb |
| Allura Red | 16035 | rot |
| 1-(4-Sulfo-1-naphthylazo)-2-naphthol-3,6-disulfosäure | 16185 | rot |
| Acid Orange 10 | 16230 | orange |
| 1-(4-Sulfo-1-naphthylazo)-2-naphthol-6,8-disulfosäure | 16255 | rot |
| 1-(4-Sulfo-1-naphthylazo)-2-naphthol-3,6,8,-trisulfosäure | 16290 | rot |
| 8-Amino-2-phenylazo-1-naphthol-3,6-disulfosäure | 17200 | rot |
| Acid Red 1 | 18050 | rot |
| Acid Red 155 | 18130 | rot |
| Acid Yellow 121 | 18690 | gelb |
| Acid Red 180 | 18736 | rot |
| Acid Yellow 11 | 18820 | gelb |
| Acid Yellow 17 | 18965 | gelb |
| 4-(4-Sulfo-1-phenylazo)-1-(4-sulfophenyl)-5-hydroxy-phrazolon-3-carbonsäure | 19140 | gelb |
| Pigment Yellow 16 | 20040 | gelb |
| 2,6-(4'-Sulfo-2",4"-dimethyl)-bis-phenylazo)1,3-dihydroxybenzol | 20170 | orange |
| Acid Black 1 | 20470 | schwarz |
| Pigment Yellow 13 | 21100 | gelb |
| Pigment Yellow 83 | 21108 | gelb |
| Solvent Yellow | 21230 | gelb |
| Acid Red 163 | 24790 | rot |
| Acid Red 73 | 27290 | rot |
| 2-[4'-(4"Sulfo-1"-phenylazo)-7'-sulfo-1'-naphthylazo]-1-hydroxy-7-aminoaphthalin-3,6-disulfosäure | 27755 | schwarz |
| 4'-[(4"-Sulfo-1"-phenylazo)-7'-sulfo-1'-naphthylazo]-1-hydroxy-8-acetyl-aminonaphthalin-3,5-disulfosäure | 28440 | schwarz |
| Direct Orange 34, 39, 44, 46, 60 | 40215 | orange |
| Food Yellow | 40800 | orange |
| Trans-ß-Apo-8'-Carotinaldehyd (C₃₀) | 40820 | orange |
| Trans-Apo-8'-Carotinsäure (C₃₀)-ethylester | 40825 | orange |
| Canthaxanthin | 40850 | orange |
| Acid Blue 1 | 42045 | blau |
| 2,4-Disulfo-5-hydroxy-4'-4"-bis-(diethylamino)triphenyl-carbinol | 42051 | blau |
| 4-[(-4-N-Ethyl-p-sulfobenzylamino)-phenyl-(4-hydroxy-2-sulfophenyl)-(methylen)-1-(N-ethylN-p-sulfobenzyl)-2,5-cyclohexadienimin] | 42053 | grün |
| Acid Blue 7 | 42080 | blau |
| (N-Ethyl-p-sulfobenzyl-amino-phenyl-(2-sulfophenyl)-methylen-(N-ethyl-N-p-sulfo-benzyl)-cyclohexadienimin | 42090 | blau |
| Acid Green 9 | 42100 | grün |
| Diethyl-di-sulfobenzyl-di-4-amino-2-chlor-di-2-methyl-fuchsonimonium | 42170 | grün |
| Basic Violet 14 | 42510 | violett |
| Basic Violet 2 | 42520 | violett |
| 2'-Methyl-4'-(N-ethyl-N-m-sulfobenzyl)-amino-4"-(N-diethyl)-amino-2-methyl-N-ethyl-N-m-sulfobenzyl-fuchsonimmonium | 42735 | blau |
| 4'-(N-Dimethyl)-amino-4"-(N-phenyl)-aminonaphtho-N-dimethyl-fuchsonimmonium | 44045 | blau |
| 2-Hydroxy-3,6-disulfo-4,4'-bis-dimethylaminonaphtofuchsinimmonium | 44090 | grün |
| Acid red | 45100 | rot |
| 3-(2'-Methylphenylamino)-6-(2'-methyl-4'-sulfophenylamino)-9-(2"-carboxyphenyl)-xantheniumsalz | 45190 | violett |
| Acid Red 50 | 45220 | rot |
| Phenyl-2-oxyfluoron-2-carbonsäure | 45350 | gelb |
| 4,5-Dibromfluorescein | 45370 | orange |
| 2,4,5,7-Tetrabromfluorescein | 45380 | rot |
| Solvent Dye | 45396 | orange |
| Acid Red 98 | 45405 | rot |
| 3',4',5',6'-Tetrachlor-2,4,5,7-tetrabromfluorescein | 45410 | rot |
| 4,5-Diiodfluorescein | 45425 | rot |
| 2,4,5,7-Tetraiodfluorescein | 45430 | rot |
| Chinophthalon | 47000 | gelb |
| Chinophthalon-disulfosäure | 47005 | gelb |
| Acid violet 50 | 50325 | violett |
| Acid Black 2 | 50420 | schwarz |
| Pigment Violet 23 | 51319 | violett |
| 1,2-Dioxyanthrachinon, Calcium-Aluminiumkomplex | 58000 | rot |
| 3-Oxypyren-5,8,10-sulfosäure | 59040 | grün |
| 1-Hydroxy-4-N-phenyl-aminoanthrachinon | 60724 | violett |
| 1-Hydroxy-4-(4'-methylphenylamino)-anthrachinon | 60725 | violett |
| Acid Violet 23 | 60730 | violett |
| 1,4-Di(4'-methyl-phenylamino)-anthrachinon | 61565 | grün |
| 1,4-Bis-(o-sulfo-p-toluidine)-anthrachinon | 61570 | grün |
| Acid Blue 80 | 61585 | blau |
| Acid Blue 62 | 62045 | blau |
| N,N'-Dihydro-1,2,1',2'-anthrachinonazin | 69800 | blau |
| Vat Blue 6; Pigment Blue 64 | 69825 | blau |
| Vat Orange 7 | 71105 | orange |
| Indigo | 73000 | blau |
| Indigo-disulfosäure | 73015 | blau |
| 4,4'-Dimethyl-6,6'-dichlorthioindigo | 73360 | rot |
| 5,5'-Dichlor-7,7'-dimethylthioindigo | 73385 | violett |
| Quinacridone Violet 19 | 73900 | violett |
| Pigment Red 122 | 73915 | rot |
| Pigment Blue 16 | 74100 | blau |
| Phthalocyanine | 74160 | blau |
| Direct Blue 86 | 74180 | blau |
| chlorierte Phthalocyanine | 74260 | grün |
| Natural Yellow 6,19; Natural Red 1 | 75100 | gelb |
| Bixin, Nor-Bixin | 75120 | orange |
| Lycopin | 75125 | gelb |
| Trans-alpha, beta- bzw. gamma-Carotin | 75130 | orange |
| Keto- und/oder Hydroxylderivate des Carotins | 75135 | gelb |
| Chemische oder sonstige Bezeichnung | CIN | Farbe |
| Guanin oder Perlglanzmittel | 75170 | weiß |
| 1,7-Bis-(4-hydroxy-3-methoxyphenyl)1,6-heptadien-3,5-dion | 75300 | gelb |
| Komplexsalz (Na,Al,Ca) der Karminsäure | 75470 | rot |
| Chlorophyll a und b; Kupferverbindungen der Chlorophylle und Chlorophylline | 75810 | grün |
| Aluminium | 77000 | weiß |
| Tonerdehydrat | 77002 | weiß |
| wasserhaltige Aluminiumsilikate | 77004 | weiß |
| Ultramarin | 77007 | blau |
| Pigment Red 101 und 102 | 77015 | rot |
| Bariumsulfat | 77120 | weiß |
| Bismutoxychlorid und seine Gemische mit Glimmer | 77163 | weiß |
| Calciumcarbonat | 77220 | weiß |
| Calciumsulfat | 77231 | weiß |
| Kohlenstoff | 77266 | schwarz |
| Pigment Black 9 | 77267 | schwarz |
| Carbo medicinalis vegetabilis | 77268:1 | schwarz |
| Chromoxid | 77288 | grün |
| Chromoxid, wasserhaltig | 77289 | grün |
| Pigment Blue 28, Pigment Green 14 | 77346 | grün |
| Pigment Metal 2 | 77400 | braun |
| Gold | 77480 | braun |
| Eisenoxide und -hydroxide | 77489 | orange |
| Eisenoxide und -hydroxide | 77491 | rot |
| Chemische oder sonstige Bezeichnung | CIN | Farbe |
| Eisenoxidhydrat | 77492 | gelb |
| Eisenoxid | 77499 | schwarz |
| Mischungen aus Eisen(II)- und Eisen(III)-hexacyanoferrat | 77510 | blau |
| Pigment White 18 | 77713 | weiß |
| Mangananimoniumdiphosphat | 77742 | violett |
| Manganphosphat; Mn₃(PO₄)₂*7H₂O | 77745 | rot |
| Silber | 77820 | weiß |
| Titandioxid und seine Gemische mit Glimmer | 77891 | weiß |
| Zinkoxid | 77947 | weiß |
| 6,7-Dimethyl-9-(1'-D-ribityl)-isoalloxazin, Lactoflavin | | gelb |
| Zuckerkulör | | braun |
| Capsanthin, Capsorubin | | orange |
| Betanin | | rot |
| Benzopyriliumsalze, Anthocyanine | | rot |
| Aluminium-, Zink-, Magnesium-, und Calciumstearat | | weiß |
| Bromthymolblau | | blau |
| Bromkresolgrün | | grün |
| Acid Red 195 | | rot |

Ferner vorteilhaft sind öllösliche Naturfarbstoffe, wie z. B. Paprikaextrakte, β-Carotin und Cochenille.

Als Duft- bzw. Parfümöle können einzelne Riechstoffverbindungen, z. B. die synthetischen Produkte vom Typ der Ester, Ether, Aldehyde, Ketone, Alkohole und Kohlenwasserstoffe verwendet werden. Riechstoffverbindungen vom Typ der Ester sind z. B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z. B. die linearen Alkanale mit 8 bis 18 C-Atomen, Citral, Citronellal, Citronellyloxyacetaldehyd, Cycllamenaldehyd, Hydroxycitronellal, Lilial und Bourgeonal, zu den Ketonen z. B. die lonone, alpha-Isomethylionon und Methyl-cedrylketon, zu den Alkoholen Anethol, Citronellol, Eugenol, Geranion, Linalol, Phenylethylalkohol und Terpineol, zu den Kohlenwasserstoffen gehören hauptsächlich die Terpene und Balsame. Bevorzugt werden Mischungen verschiedener Riechstoffe verwendet, die gemeinsam eine ansprechende Duftnote erzeugen.

Parfümöle können auch natürliche Riechstoffgemische enthalten, wie sie aus pflanzlichen oder tierischen Quellen zugänglich sind, z. B. Pinien-, Citrus-, Jasmin-, Lilien-, Rosen-, oder Ylang-Ylang-Öl. Auch ätherische Öle geringerer Flüchtigkeit, die meist als Aromakomponenten verwendet werden, eignen sich als Parfümöle, z. B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl und Ladanumöl.

Als perlglanzgebende Komponente bevorzugt geeignet sind

Fettsäuremonoalkanolamide, Fettsäuredialkanolamide, Monoester oder Diester von Alkylenglykolen, insbesondere Ethylenglykol und/oder Propylenglykol oder dessen Oligomere, mit höheren Fettsäuren, wie z. B. Palmitinsäure, Stearinsäure und Behensäure, Monoester oder Polyester von Glycerin mit Carbonsäuren, Fettsäuren und deren Metallsalze, Ketosulfone oder Gemische der genannten Verbindungen. Besonders bevorzugt sind Ethylenglykoldistearate und/oder Polyethylenglykol-distearate mit durchschnittlich 3 Glykoleinheiten.

Sofern die erfindungsgemäßen Zusammensetzungen perlglanzgebende Verbindungen enthalten, sind diese bevorzugt in einer Menge von 0,1 bis 15,0 Gew.-% und besonders bevorzugt in einer Menge von 1,0 bis 10,0 Gew.-% in den erfindungsgemäßen Zusammensetzungen enthalten, bezogen auf das Gesamtgewicht der Zusammensetzungen.

Als Säuren oder Laugen zur pH-Wert Einstellung werden vorzugsweise Mineralsäuren, insbesondere HCl, anorganische Basen, insbesondere NaOH oder KOH, und organische Säuren, insbesondere Zitronensäure, verwendet.

Vorzugsweise weisen die erfindungsgemäßen Zusammensetzungen einen pH-Wert von 2 bis 10, besonders bevorzugt von 2 bis 7 und insbesondere bevorzugt von 2,5 bis 4,5 auf.

In einer weiteren bevorzugten Ausführungsform der Erfindung handelt es sich bei den erfindungsgemäßen Zusammensetzungen um Zusammensetzungen zum Bleichen und/oder Färben von Haaren oder um eine Dauerwellformulierung.

In einer weiteren bevorzugten Ausführungsform der Erfindung handelt es sich bei den erfindungsgemäßen Zusammensetzungen um oxidative Reinigerformulierungen.

In einer weiteren bevorzugten Ausführungsform der Erfindung handelt es sich bei den erfindungsgemäßen Zusammensetzungen um Fleckengele zur Vorbehandlung von Wäsche, Vorwaschsprays, Fleckentferner, Oberflächenreiniger, Toilettenreiniger, Multifunktionsfleckengele oder Bleichgele.

In einer weiteren bevorzugten Ausführungsform der Erfindung handelt es sich bei den erfindungsgemäßen Zusammensetzungen um Haarfärbezusammensetzungen zur Verhinderung von Bleichschäden bei der Haarfärbung.

Hydroxypyridone und deren Salze sind in vorteilhafter Weise zur Stabilisierung eines oder mehrerer Polymere mit verdickenden Eigenschaften geeignet. Ein weiterer Gegenstand der vorliegenden Erfindung betrifft daher die Verwendung einer oder mehrerer Substanzen ausgewählt aus der Gruppe bestehend aus Hydroxypyridonen und deren Salzen zur Stabilisierung eines oder mehrerer Polymere mit verdickenden Eigenschaften, vorzugsweise in einer erfindungsgemäßen Zusammensetzung. Dabei handelt es sich bei den erfindungsgemäßen Zusammensetzungen vorzugsweise um Zusammensetzungen zum Bleichen und/oder Färben von Haaren, um Dauerwellformulierungen oder um oxidative Reinigerformulierungen.

Vorzugsweise ist das eine oder sind die mehreren zu stabilisierenden Polymere mit verdickenden Eigenschaften ausgewählt aus teilweise oder vollständig neutralisierten, sulfonsäuregruppenhaltigen Polymeren.

Hierbei ist das eine oder sind die mehreren teilweise oder vollständig neutralisierten, sulfonsäuregruppenhaltigen Polymere vorzugsweise ausgewählt aus der Gruppe bestehend aus Homo- oder Copolymeren von Acrylamidomethylpropansulfonsäue und deren Salzen.

Bei den Hydroxypyridonen und deren Salzen, die wie soeben beschrieben erfindungsgemäß zur Stabilisierung verwendet werden können, handelt es sich um die im Rahmen der Beschreibung der erfindungsgemäßen Zusammensetzungen genannten Verbindungen der Formel (I) und ihrer Salze. Vorzugsweise werden diejenigen Verbindungen der Formel (I) und ihrer Salze zur Stabilisierung verwendet, die auch bei der Beschreibung der erfindungsgemäßen Zusammensetzungen als bevorzugt aufgeführt worden sind.

Die nachfolgenden Beispiele und Anwendungen sollen die Erfindung näher erläutern, ohne sie jedoch darauf zu beschränken. Bei allen Prozentangaben handelt es sich um Gewichts-% (Gew.-%), sofern nicht explizit anders angegeben.

### Versuchsbeispiele:

### Beispiel 1: Wasserstoffperoxid-Gel verdickt mit einem Assoziativverdicker Rezeptur:

| | |
|---|---|
| Wasser, vollentsalzt | ad 100 Gew.-% |
| Wasserstoffperoxidlösung (Solvay, 35 Gew.-%, wässrig) | 17 Gew.-% |
| Triceteareth-50 Phosphate | 6 Gew.-% |
| (Substanz nach Beispiel 1 aus DE 10 2008 006858) | |
| 4-Methyl-6-(2,4,4-trimethylpentyl)-1-hydroxy-2-pyridon | 0,00077 Gew.-% |

Die Formulierung wurde durch Lösen des polymeren Verdickers in Wasser, anschließendes Einmischen der Wasserstoffperoxidlösung und Einstellung mit 10 Gew.-%iger wässriger Phosphorsäure auf den jeweiligen Start-pH hergestellt. Die Formulierung wurde jeweils mit und ohne Zugabe von 7,7 ppm (0,00077 Gew.-%) 4-Methyl-6-(2,4,4-trimethylpentyl)-1-hydroxy-2-pyridon hergestellt. Hierzu wurden 10 ml einer 0,1 Gew.%igen Lösung des Stabilisators in Propylenglykol in 1 Liter Wasser gelöst und diese Lösung als Wasserphase eingesetzt. Ein Blindversuch mit Propylenglykol schloss einen Einfluss des Lösungsmittels aus. Die Ergebnisse von Viskositätsmessungen sind in Tabelle 1 dargestellt.

**Tabelle 1 Ergebnisse von Viskositätsmessungen**

| Start-pH | 7,7 ppm 4-Methyl-6-(2,4,4-trimethylpentyl)-1-hydroxy2-pyridon | Viskosität [mPa · s] sofort | Viskosität [mPa · s] nach 4 Wochen 40 °C | Viskosität [mPa · s] nach 8 Wochen 40 °C | Viskosität [mPa · s] nach 12 Wochen 40 °C |
|---|---|---|---|---|---|
| 3,9 | nein | 12 000 | 8 000 | 4 070 | 690 |
| 3,3 | nein | 10 400 | 1 600 | 715 | 207 |
| 3,9 | ja | 13 400 | 9 100 | 7 850 | 5350 |
| 3,2 | ja | 11 200 | 8 150 | 7 300 | 5550 |

Gleichzeitig wurde jeweils der Wasserstoffperoxid-Gehalt iodometrisch bestimmt. Über die gesamte Lagerzeit blieb dieser konstant bei 6,2 - 6,7 Gew.-%.

Das Versuchsergebnis zeigt, dass die Zugabe des Stabilisators 4-Methyl-6-(2,4,4-trimethylpentyl)-1-hydroxy-2-pyridon gegenüber einer unstabilisierten Probe einen deutlich verringerten Viskositätsabfall zeigt und damit die Stabilität des Verdickerpolymers signifikant erhöht.

### Beispiel 2: Wasserstoffperoxid-Gel verdickt mit einem Sulfonat-Copoylmer Rezeptur:

| | |
|---|---|
| Wasser, vollentsalzt | ad 100 Gew.-% |
| Wasserstoffperoxidlösung (Solvay, 35 Gew.-%, wässrig) | 17 Gew.-% |
| Aristoflex^{®} TAC | 1,5 Gew.-% |
| (Ammonium Acryloyl Dimethyltaurate / Carboxyethyl Acrylate Crosspolymer) | |
| 4-Methyl-6-(2,4,4-trimethylpentyl)-1-hydroxy-2-pyridon | 0,00088 Gew.-% |

Die Formulierung wurde durch Lösen des polymeren Verdickers in Wasser und anschließendes Einmischen der Wasserstoffperoxidlösung hergestellt. Der jeweilige Start-pH-Wert wurde anschließend mit 10 Gew.-%iger wässriger Phosphorsäure eingestellt.

Die Formulierung wurde jeweils mit und ohne Zugabe von 8,8 ppm (0,00088 Gew.-%) 4-Methyl-6-(2,4,4-trimethylpentyl)-1-hydroxy-2-pyridon hergestellt.
Hierzu wurden 10 ml einer 0,1 Gew.-%igen Lösung des Stabilisators in Propylenglykol in 1 Liter Wasser gelöst und diese Lösung als Wasserphase eingesetzt. Ein Blindversuch mit Propylenglykol schloss einen Einfluss des Lösungsmittels aus. Die Ergebnisse von Viskositätsmessungen sind in Tabelle 2 dargestellt.

**Tabelle 2 Ergebnisse von Viskositätsmessungen**

| Start-pH | 8,8 ppm 4-Methyl-6-(2,4,4-trimethylpentyl)-1-hydroxy2-pyridon | Viskosität [mPa · s] sofort | Viskosität [mPa · s] nach 4 Wochen 40 °C | Viskosität [mPa · s] nach 8 Wochen 40 °C | Viskosität [mPa · s] nach 12 Wochen 40 °C |
|---|---|---|---|---|---|
| 4,0 | nein | 3 000 | 408 | 10 | 10 |
| 3,2 | nein | 2 900 | 590 | 10 | 10 |
| 4,1 | ja | 3 050 | 2 100 | 2 530 | 2 280 |
| 3,49 | ja | 2 870 | 2 440 | 2 300 | 2 070 |

Gleichzeitig wurde jeweils der Wasserstoffperoxid-Gehalt iodometrisch bestimmt. Über die gesamte Lagerzeit blieb dieser nahezu konstant bei 5,8 - 6,3 Gew.-%.

Das Versuchsergebnis zeigt, dass die Zugabe des Stabilisators 4-Methyl-6-(2,4,4-trimethylpentyl)-1-hydroxy-2-pyridon gegenüber einer unstabilisierten Probe einen deutlich verringerten Viskositätsabfall zeigt und damit die Stabilität des Verdickerpolymers signifikant erhöht.

### Beispiel 3: Wasserstoffperoxid-Gel verdickt mit einem Sulfonat-Copolymer Rezeptur:

| | |
|---|---|
| Wasser, vollentsalzt | ad 100 Gew.-% |
| Wasserstoffperoxidlösung (Evonik, 50 Gew.-%ig, wässrig) | 12,14 Gew.-% |
| Aristoflex^{®} AVS | 0,5 Gew.-% |
| Sodium AcryloyldimethyltaurateV/P Crosspolymer | |
| 4-Methyl-6-(2,4,4-trimethylpentyl)-1-hydroxy-2-pyridon | 0,00087 Gew.-% |

Die Formulierung wurde durch Lösen des polymeren Verdickers in Wasser und anschließendes Einmischen der Wasserstoffperoxidlösung hergestellt. Der jeweilige Start-pH-Wert wurde anschließend mit 10 Gew.-%iger wässriger Phosphorsäure eingestellt. Die Formulierung wurde jeweils mit und ohne Zugabe von 8,7 ppm (0,00087 Gew.-%) 4-Methyl-6-(2,4,4-trimethylpentyl)-1-hydroxy-2-pyridon hergestellt.
Hierzu wurden 10 ml einer 0,1 Gew.%igen Lösung des Stabilisators in Propylenglykol in 1 Liter Wasser gelöst und diese Lösung als Wasserphase eingesetzt. Ein Blindversuch mit Propylenglykol schloss einen Einfluss des Lösungsmittels aus. Die Ergebnisse von Viskositätsmessungen sind in Tabelle 3 dargestellt.

**Tabelle 3 Ergebnisse von Viskositätsmessungen**

| Start-pH | 8,7 ppm (= 0,00087 Gew.-%) 4-Methyl-6-(2,4,4-trimethylpentyl)-1-hydroxy-2-pyridon | Viskosität [mPa · s] sofort | Viskosität [mPa · s] nach 4 Wochen 40 °C |
|---|---|---|---|
| 3,90 | nein | 12 700 | 10 |
| 3,42 | nein | 10 600 | 10 |
| 3,9 | ja | 15 700 | 16 000 |
| 3,29 | ja | 10 200 | 8 800 |

Gleichzeitig wurde jeweils der Wasserstoffperoxid-Gehalt iodometrisch bestimmt. Über die gesamte Lagerzeit blieb dieser nahezu konstant bei 5,8 - 6,3 Gew.-%.

Das Versuchsergebnis zeigt, dass die Zugabe des Stabilisators 4-Methyl-6-(2,4,4-trimethylpentyl)-1-hydroxy-2-pyridon gegenüber einer unstabilisierten Probe einen deutlich verringerten Viskositätsabfall zeigt und damit die Stabilität des Verdickerpolymers signifikant erhöht.

Wasserstoffperoxid wurde von Solvay (35 Gew.-%ig, wässrig, Interox^{®} AG-CO-35) bzw. Evonik (50 Gew.-%ig, wässrig, Quality B7) bezogen.

Die Viskositäten wurden mit einem Brookfield-Viskosimeter Model DV II, den Spindeln aus dem Spindelset RV bei 20 Umdrehungen / Minute und 20 °C gemessen. Es werden die Spindeln 1 bis 7 aus dem Spindelset RV verwendet. Unter diesen Messbedingungen wird Spindel 1 für Viskositäten von maximal 500 mPa · s, Spindel 2 für Viskositäten von maximal 1 000 mPa · s, Spindel 3 für Viskositäten von maximal 5 000 mPa · s, Spindel 4 für Viskositäten von maximal 10 000 mPa · s, Spindel 5 für Viskositäten von maximal 20 000 mPa · s, Spindel 6 für Viskositäten von maximal 50 000 mPa · s und Spindel 7 für Viskositäten von maximal 200 000 mPa · s gewählt.

### Beispiel 4: Wasserstoffperoxid-Gel verdickt mit einem Sulfonat-Copolymer Rezeptur:

| | |
|---|---|
| Wasser, vollentsalzt | ad 100 Gew.-% |
| Wasserstoffperoxidlösung (Evonik, 50 Gew.-%ig, wässrig) | 12,14 Gew.-% |
| Aristoflex^{®} AVS | 0,5 Gew.-% |
| Sodium Acryloyldimethyltaurate/VP Crosspolymer 1-Hydroxy-2-pyridon | 0,001 Gew.-% |

Die Formulierung wurde durch Lösen des polymeren Verdickers in Wasser und anschließendes Einmischen der Wasserstoffperoxidlösung hergestellt. Der jeweilige Start-pH-Wert wurde anschließend mit 10 Gew.-%iger wässriger Phosphorsäure eingestellt. Die Formulierung wurde jeweils mit und ohne Zugabe von 10 ppm (0,001 Gew.-%) 1-Hydroxy-2-pyridon hergestellt. Hierzu wurden 10 ml einer 0,1 Gew.%igen Lösung des Stabilisators in Propylenglykol in 1 Liter Wasser gelöst und diese Lösung als Wasserphase eingesetzt. Ein Blindversuch mit Propylenglykol schloss einen Einfluss des Lösungsmittels aus. Die Ergebnisse von Viskositätsmessungen sind in Tabelle 4 dargestellt.

**Tabelle 4 Ergebnisse von Viskositätsmessungen**

| Start-pH | 10 ppm (= 0,001 Gew.-%) 4- 1-Hydroxy-2-pyridon | Viskosität [mPa · s] sofort | Viskosität [mPa · s] nach 4 Wochen 40 °C |
|---|---|---|---|
| 4,0 | nein | 5 850 | 10 |
| 3,0 | nein | 5 200 | 10 |
| 4,0 | ja | 6 700 | 6 100 |
| 3,0 | ja | 5 900 | 5 500 |

Gleichzeitig wurde jeweils der Wasserstoffperoxid-Gehalt iodometrisch bestimmt. Über die gesamte Lagerzeit blieb dieser nahezu konstant bei 5,8 - 6,3 Gew.-%.

Das Versuchsergebnis zeigt, dass die Zugabe des Stabilisators 1-Hydroxy-2-pyridon gegenüber einer unstabilisierten Probe einen deutlich verringerten Viskositätsabfall zeigt und damit die Stabilität des Verdickerpolymers signifikant erhöht.

### Beispiel 5: Wasserstoffperoxid-Gel verdickt mit einem Biopolymer Rezeptur:

| | |
|---|---|
| Wasser, vollentsalzt | ad 100 Gew.-% |
| Wasserstoffperoxidlösung (Solvay, 35 Gew.%ig, wässrig) | 17 Gew.-% |
| Xanthan Gum | 1,5 Gew-% |
| 4-Methyl-6-(2,4,4-trimethylpentyl)-1-hydroxy-2-pyridon | 0,00081 Gew.-% |

Die Formulierung wurde durch Lösen des polymeren Verdickers in Wasser und anschließendes Einmischen der Wasserstoffperoxidlösung hergestellt. Der jeweilige Start-pH-Wert wurde anschließend mit 10 Gew.-%iger wässriger Phosphorsäure eingestellt. Die Formulierung wurde jeweils mit und ohne Zugabe von 8,1 ppm (0,00081 Gew.-%) 4-Methyl-6-(2,4,4-trimethylpentyl)-1-hydroxy-2-pyridon hergestellt.
Hierzu wurden 10 ml einer 0,1 Gew.%igen Lösung des Stabilisators in Propylenglykol in 1 Liter Wasser gelöst und diese Lösung als Wasserphase eingesetzt. Ein Blindversuch mit Propylenglykol schloss einen Einfluss des Lösungsmittels aus. Die Ergebnisse von Viskositätsmessungen sind in Tabelle 5 dargestellt.

**Tabelle 5 Ergebnisse von Viskositätsmessungen**

| Start-pH | 8,1 ppm (= 0,00081 Gew.-%) 4-Methyl-6-(2,4,4-trimethylpentyl)-1-hydroxy-2-pyridon | Viskosität [mPa · s] sofort | Viskosität [mPa · s] nach 4 Wochen 40 °C |
|---|---|---|---|
| 4,0 | nein | 3 500 | 10 |
| 3,0 | nein | 5 000 | 10 |
| 4,0 | ja | 4 000 | 4 150 |
| 3,0 | ja | 4 800 | 4 700 |

Gleichzeitig wurde jeweils der Wasserstoffperoxid-Gehalt iodometrisch bestimmt. Über die gesamte Lagerzeit blieb dieser nahezu konstant bei 5,8 - 6,3 Gew.-%.

Das Versuchsergebnis zeigt, dass die Zugabe des Stabilisators 4-Methyl-6-(2,4,4-trimethylpentyl)-1-hydroxy-2-pyridon gegenüber einer unstabilisierten Probe einen deutlich verringerten Viskositätsabfall zeigt und damit die Stabilität des Verdickerpolymers signifikant erhöht.

### Beispiel 6: Wasserstoffperoxid-Gel verdickt mit einem Biopolymer Rezeptur:

| | |
|---|---|
| Wasser, vollentsalzt | ad 100 Gew.-% |
| Wasserstoffperoxidlösung (Solvay, 35 Gew.-%ig, wässrig) | 17 Gew.-% |
| Hydroxyethylcellulose | 2,0 Gew-% |
| 4-Methyl-6-(2,4,4-trimethylpentyl)-1-hydroxy-2-pyridon | 0,00081 Gew.-% |

Die Formulierung wurde durch Lösen des polymeren Verdickers in Wasser und anschließendes Einmischen der Wasserstoffperoxidlösung hergestellt. Der jeweilige Start-pH-Wert wurde anschließend mit 10 Gew.-%iger wässriger Phosphorsäure eingestellt. Die Formulierung wurde jeweils mit und ohne Zugabe von 8,1 ppm (0,00081 Gew.-%) 4-Methyl-6-(2,4,4-trimethylpentyl)-1-hydroxy-2-pyridon hergestellt.
Hierzu wurden 10 ml einer 0,1 Gew.-%igen Lösung des Stabilisators in Propylenglykol in 1 Liter Wasser gelöst und diese Lösung als Wasserphase eingesetzt. Ein Blindversuch mit Propylenglykol schloss einen Einfluss des Lösungsmittels aus. Die Ergebnisse von Viskositätsmessungen sind in Tabelle 6 dargestellt.

**Tabelle 6 Ergebnisse von Viskositätsmessungen**

| Start-pH | 8,1 ppm (= 0,00081 Gew.-%) 4-Methyl-6-(2,4,4-trimethylpentyl)-1-hydroxy-2-pyridon | Viskosität [mPa · s] nach 10 Stunden | Viskosität [mPa · s] nach 4 Wochen 40 °C |
|---|---|---|---|
| 4,0 | nein | 530 | 10 |
| 3,0 | nein | 575 | 10 |
| 4,0 | ja | 9 850 | 5 200 |
| 3,0 | ja | 9 800 | 4 360 |

Gleichzeitig wurde jeweils der Wasserstoffperoxid-Gehalt iodometrisch bestimmt. Über die gesamte Lagerzeit blieb dieser nahezu konstant bei 5,8 - 6,3 Gew.-%.

Das Versuchsergebnis zeigt, dass die Zugabe des Stabilisators 4-Methyl-6-(2,4,4-trimethylpentyl)-1-hydroxy-2-pyridon gegenüber einer unstabilisierten Probe einen deutlich verringerten Viskositätsabfall zeigt und damit die Stabilität des Verdickerpolymers signifikant erhöht.

### Beispiel 7: Wasserstoffperoxid-Gel verdickt mit einem Polyalkylenglykol Rezeptur:

| | |
|---|---|
| Wasser, vollentsalzt | ad 100 Gew.-% |
| Wasserstoffperoxidlösung (Solvay, 35 Gew.-%ig, wässrig) | 17 Gew.-% |
| Polyethylenglykol (Polyox WSR-301, Dow) | 1,75 Gew-% |
| 4-Methyl-6-(2,4,4-trimethylpentyl)-1-hydroxy-2-pyridon | 0,00081 Gew.-% |

Die Formulierung wurde durch Lösen des polymeren Verdickers in Wasser und anschließendes Einmischen der Wasserstoffperoxidlösung hergestellt. Der jeweilige Start-pH-Wert wurde anschließend mit 10 Gew.%iger wässriger Phosphorsäure eingestellt. Die Formulierung wurde jeweils mit und ohne Zugabe von 8,1 ppm (0,00081 Gew.-%) 4-Methyl-6-(2,4,4-trimethylpentyl)-1-hydroxy-2-pyridon hergestellt.
Hierzu wurden 10 ml einer 0,1 Gew.-%igen Lösung des Stabilisators in Propylenglykol in 1 Liter Wasser gelöst und diese Lösung als Wasserphase eingesetzt. Ein Blindversuch mit Propylenglykol schloss einen Einfluss des Lösungsmittels aus. Die Ergebnisse von Viskositätsmessungen sind in Tabelle 7 dargestellt.

**Tabelle 7 Ergebnisse von Viskositätsmessungen**

| Start-pH | 8,1 ppm (= 0,00081 Gew.-%) 4-Methyl-6-(2,4,4-trimethylpentyl)-1-hydroxy-2-pyridon | Viskosität [mPa · s] sofort | Viskosität [mPa · s] nach 1 Woche 25 °C |
|---|---|---|---|
| 4,0 | nein | 2 560 | 50 |
| 3,0 | nein | 2 490 | 60 |
| 4,0 | ja | 2 920 | 2 100 |
| 3,0 | ja | 3 080 | 2 050 |

Gleichzeitig wurde jeweils der Wasserstoffperoxid-Gehalt iodometrisch bestimmt. Über die gesamte Lagerzeit blieb dieser nahezu konstant bei 5,8 - 6,3 Gew.-%.

Das Versuchsergebnis zeigt, dass die Zugabe des Stabilisators 4-Methyl-6-(2,4,4-trimethylpentyl)-1-hydroxy-2-pyridon gegenüber einer unstabilisierten Probe einen deutlich verringerten Viskositätsabfall zeigt und damit die Stabilität des Verdickerpolymers signifikant erhöht.

### Beispiel 8: Wasserstoffperoxid-Gel verdickt mit einem Sulfonat-Copolymer Rezeptur:

| | |
|---|---|
| Wasser, vollentsalzt | ad 100 Gew.-% |
| Wasserstoffperoxidlösung (Solvay, 35 Gew.-%ig, wässrig) | 17 Gew.-% |
| Aristoflex^{®} HMS | 0,5 Gew-% |
| Ammonium Acryloyldimethyltaurate/Steareth-25 Crosspolymer 4-Methyl-6-(2,4,4-trimethylpentyl)-1-hydroxy-2-pyridon | 0,00081 Gew.-% |

Die Formulierung wurde durch Lösen des polymeren Verdickers in Wasser und anschließendes Einmischen der Wasserstoffperoxidlösung hergestellt. Der jeweilige Start-pH-Wert wurde anschließend mit 10 Gew.-%iger wässriger Phosphorsäure eingestellt. Die Formulierung wurde jeweils mit und ohne Zugabe von 8,1 ppm (0,00081 Gew.-%) 4-Methyl-6-(2,4,4-trimethylpentyl)-1-hydroxy-2-pyridon hergestellt.
Hierzu wurden 10 ml einer 0,1 Gew.-%igen Lösung des Stabilisators in Propylenglykol in 1 Liter Wasser gelöst und diese Lösung als Wasserphase eingesetzt. Ein Blindversuch mit Propylenglykol schloss einen Einfluss des Lösungsmittels aus. Die Ergebnisse von Viskositätsmessungen sind in Tabelle 8 dargestellt.

**Tabelle 8 Ergebnisse von Viskositätsmessungen**

| Start-pH | 8,1 ppm (= 0,00081 Gew.-%) 4-Methyl-6-(2,4,4-trimethylpentyl)-1-hydroxy-2-pyridon | Viskosität [mPa · s] sofort | Viskosität [mPa · s] nach 8 Wochen 40 °C |
|---|---|---|---|
| 4,0 | nein | 3 320 | 205 |
| 3,0 | nein | 2 760 | 745 |
| 4,0 | ja | 3 350 | 6 100 |
| 3,0 | ja | 2 740 | 6 000 |

Gleichzeitig wurde jeweils der Wasserstoffperoxid-Gehalt iodometrisch bestimmt. Über die gesamte Lagerzeit blieb dieser nahezu konstant bei 5,8 - 6,3 Gew.-%.

Das Versuchsergebnis zeigt, dass die Zugabe des Stabilisators 4-Methyl-6-(2,4,4-trimethylpentyl)-1-hydroxy-2-pyridon gegenüber einer unstabilisierten Probe einen deutlich verringerten Viskositätsabfall zeigt und damit die Stabilität des Verdickerpolymers signifikant erhöht.

### Beispiel 9: Wasserstoffperoxid-Gel verdickt mit einem Sulfonat-Polymer Rezeptur:

| | |
|---|---|
| Wasser, vollentsalzt | ad 100 Gew.-% |
| Wasserstoffperoxidlösung (Solvay, 35 Gew.-%ig, wässrig) | 17 Gew.-% |
| Aristoflex^{®} AVS | 1,0 Gew-% |
| Sodium Acryloyldimethyltaurate/VP Crosspolymer | |
| 4-Methyl-6-(2,4,4-trimethylpentyl)-1-hydroxy-2-pyridon | 0,00081 Gew.-% |

Die Formulierung wurde durch Lösen des polymeren Verdickers in Wasser und anschließendes Einmischen der Wasserstoffperoxidlösung hergestellt. Der jeweilige Start-pH-Wert wurde anschließend mit 10 Gew.-%iger wässriger Natronlauge eingestellt. Die Formulierung wurde jeweils mit und ohne Zugabe von 8,1 ppm (0,00081 Gew.-%) 4-Methyl-6-(2,4,4-trimethylpentyl)-1-hydroxy-2-pyridon hergestellt.
Hierzu wurden 10 ml einer 0,1 Gew.-%igen Lösung des Stabilisators in Propylenglykol in 1 Liter Wasser gelöst und diese Lösung als Wasserphase eingesetzt. Ein Blindversuch mit Propylenglykol schloss einen Einfluss des Lösungsmittels aus. Die Ergebnisse von Viskositätsmessungen sind in Tabelle 9 dargestellt.

**Tabelle 9 Ergebnisse von Viskositätsmessungen**

| Start-pH | 8,1 ppm (= 0,00081 Gew.-%) 4-Methyl-6-(2,4,4-trimethylpentyl)-1-hydroxy-2-pyridon | Viskosität [mPa · s] sofort | Viskosität [mPa · s] nach 2 Wochen 25 °C |
|---|---|---|---|
| 9,0 | nein | 4 450 | 10 |
| 9,0 | ja | 6 550 | 7 300 |

Gleichzeitig wurde jeweils der Wasserstoffperoxid-Gehalt iodometrisch bestimmt. Vom Startwert 6,0 Gew.-% fiel der Wasserstoffperoxidgehalt der Lösung ohne Stabilisator auf 0,4 Gew.-% ab, während mit Stabilisator noch der Anfangswert von 6,0 Gew.-% erhalten war.

Das Versuchsergebnis zeigt, dass die Zugabe des Stabilisators 4-Methyl-6-(2,4,4-trimethylpentyl)-1-hydroxy-2-pyridon gegenüber einer unstabilisierten Probe einen deutlich verringerten Viskositätsabfall auch bei hohem pH-Wert zeigt und damit die Stabilität des Verdickerpolymers signifikant erhöht.

### Beispiel 10: Wasserstoffperoxid-Gel verdickt mit einem Sulfonat-Copoylmer Rezeptur:

| | |
|---|---|
| Wasser, vollentsalzt | ad 100 Gew.-% |
| Wasserstoffperoxid lösung (Solvay, 35 Gew.-%, wässrig) | 17 Gew.-% |
| Hostapur^{®} SAS (100 % active) | 4,5 Gew-% |
| Sodium C14-17 Alkyl Sec Sulfonate | |
| Aristoflex^{®} TAC | 1,5 Gew.-% |
| (Ammonium Acryloyl Dimethyltaurate / Carboxyethyl Acrylate Crosspolymer) | |
| 4-Methyl-6-(2,4,4-trimethylpentyl)-1-hydroxy-2-pyridon | 0,00088 Gew.-% |

Die Formulierung wurde durch Lösen des polymeren Verdickers in Wasser und anschließendes Einmischen der Wasserstoffperoxidlösung hergestellt. Der jeweilige Start-pH-Wert wurde anschließend mit 10 Gew.-%iger wässriger Phosphorsäure eingestellt. Die Formulierung wurde jeweils mit und ohne Zugabe von 8,8 ppm (0,00088 Gew.-%) 4-Methyl-6-(2,4,4-trimethylpentyl)-1-hydroxy-2-pyridon hergestellt.
Hierzu wurden 10 ml einer 0,1 Gew.-%igen Lösung des Stabilisators in Propylenglykol in 1 Liter Wasser gelöst und diese Lösung als Wasserphase eingesetzt. Ein Blindversuch mit Propylenglykol schloss einen Einfluss des Lösungsmittels aus. Die Ergebnisse von Viskositätsmessungen sind in Tabelle 10 dargestellt.

**Tabelle 10 Ergebnisse von Viskositätsmessungen**

| Start-pH | 8,8 ppm 4-Methyl-6-(2,4,4-trimethyl-pentyl)-1-hydroxy-2-pyridon | Viskosität [mPa · s] sofort | Viskosität [mPa · s] nach 4 Wochen 40 °C |
|---|---|---|---|
| 4,0 | Nein | 1 340 | 375 |
| 7,0 | Nein | 1 450 | 625 |
| 4,0 | Ja | 1 320 | 1 230 |
| 7,0 | Ja | 1 400 | 1 150 |

Gleichzeitig wurde jeweils der Wasserstoffperoxid-Gehalt iodometrisch bestimmt. Über die gesamte Lagerzeit blieb dieser nahezu konstant bei 5,8 - 6,3 Gew.%.

Das Versuchsergebnis zeigt, dass die Zugabe des Stabilisators 4-Methyl-6-(2,4,4-trimethylpentyl)-1-hydroxy-2-pyridon gegenüber einer unstabilisierten Probe einen deutlich verringerten Viskositätsabfall zeigt und damit die Stabilität des Verdickerpolymers signifikant erhöht.

### Formulierungsbeispiele:

### Formulierungsbeispiel 1: Emulgatorhaltige Bleichcreme

| Phase | Inhaltsstoff | Gew.-% |
|---|---|---|
| A | Montanov^{®} 68 | 1,5 |
| | Cetearyl Alcohol and Cetearyl Glucoside | |
| | Sepinov^{®} EMT 10 | 1,5 |
| | Hydroxyethyl Acrylate / Sodium Acryloyldimethyl Taurate Copolymer | |
| | Paraffin Oil | 20 |
| B | Sodium Pyrophosphate | 0,6 |
| | Wasserstoffperoxid (30 Gew.-%ig, wässrig) | 6 |
| | Konservierungsmittel | qs |
| | 4-Methyl-6-(2,4,4-trimethylpentyl)-1-hydroxy-2-pyridon, Monoethanolamin Salz (Octopirox^{®}, Clariant) | 0,002 |
| | Wasser | ad 100 |

### Herstellung:

Sepinov EMT 10 wird in der Phase A dispergiert. Die Komponenten der Phase B werden gemischt und Phase A eingerührt, wobei sich eine Emulsion bildet.

### Formulierungsbeispiel 2: Emulgatorfreies Bleichgel

| Inhaltsstoff | Gew.-% |
|---|---|
| Aristoflex^{®} AVS | 1,0 |
| Sodium Acryloyldimethyltaurate/VP Crosspolymer | |
| Wasserstoffperoxid (35 Gew.-%ig, wässrig) | 17 |
| Natriumpyrophosphat | 0,02 |
| Natriumstannat | 0,04 |
| 4-Methyl-6-(2,4,4-trimethylpentyl)-1-hydroxy-2-pyridon | 0,001 |
| Propylenglykol | 1 |
| Wasser | ad 100 |

### Herstellung:

4-Methyl-6-(2,4,4-trimethylpentyl)-1-hydroxy-2-pyridon wird in Propylenglykol gelöst. Aristoflex^{®} AVS wird in Wasser aufgelöst, sodann wird die Wasserstoffperoxidlösung eingerührt, gefolgt von den beiden Stabilisatoren.

### Formulierungsbeispiel 3: Oxidative Haarfärbeformulierung

### Färbebasis:

| Inhaltsstoffe | Gew.-% |
|---|---|
| Polyquaternium-29 | 0,5 |
| (Dihydroxyproyl Chitosan Trimonium Chloride) | |
| m-Phenylendiamin | 0,08 |
| p-Phenylendiamine HCl | 0,30 |
| Resorcinol | 0,25 |
| Sodium Bisulfite | 0,30 |
| Sodium Laureth Sulfate | 3,50 |

| Inhaltsstoff | Gew.-% |
|---|---|
| Cetyl Alcohol | 15,00 |
| Ammonia, 25 Gew.%ig, wässrig | 2,00 |
| Wasser | ad 100 |

### Herstellung:

Cetyl alcohol und Sodium Laureth Sulfate werden auf 60°C erhitzt, gemischt und unter Rühren in die Wasserphase eingetragen, in der die übrigen Inhaltsstoffe gelöst wurden.

### Entwicklergel:

| Inhaltsstoff | Gew.-% |
|---|---|
| Aristoflex^{®} TAC | 1,5 |
| Ammonium Acryloyldimethyltaurate/Carboxyethyl Acrylate Crosspolymer | |
| Wasserstoffperoxid (35 Gew.%ig, wässrig) | 18 |
| Natriumpyrophosphat | 0,02 |
| 4-Methyl-6-(cyclohexyl)-1-hydroxy-2-pyridon | 0,002 |
| Propylenglykol | 1 |
| Wasser | ad 100 |

### Herstellung:

4-Methyl-6-(cyclohexyl)-1-hydroxy-2-pyridon wird in Propylenglykol gelöst. Aristoflex^{®} TAC wird in Wasser aufgelöst, sodann wird die Wasserstoffperoxidlösung eingerührt, gefolgt vom Stabilisator Natriumpyrophosphat.

Es entsteht ein Gel mit einer Viskosität von ca. 3 000 mPa · s bei 20 °C.

### Färbeprozedur:

50 ml der Färbebasis werden mit 50 ml des Entwicklergels verrührt und auf das Haar appliziert. Nach 30 Minuten wird ausgespült.

### Formulierungsbeispiel 4: Oxidative Haarfärbeformulierung, Ammoniakfrei Färbebasis:

| Inhaltsstoff | Gew.-% |
|---|---|
| Polyquaternium-29 | 0,5 |
| (Dihydroxyproyl Chitosan Trimonium Chloride) | |
| m-Phenylendiamin | 0,08 |
| p-Phenylendiamine HCl | 0,30 |
| Resorcinol | 0,25 |
| Sodium Bisulfite | 0,30 |
| Sodium Laureth Sulfate | 3,50 |
| Cetyl Alcohol | 15,00 |
| Monoethanolamin | 2,00 |
| Wasser | ad 100 |

### Herstellung:

Cetyl alcohol und Sodium Laureth Sulfate werden auf 60°C erhitzt, gemischt und unter Rühren in die Wasserphase eingetragen, in der die übrigen Inhaltsstoffe gelöst wurden.

### Entwicklergel:

| Inhaltsstoff | Gew.-% |
|---|---|
| Aristoflex^{®} TAC | 1,5 |
| Ammonium Acryloyldimethyltaurate/Carboxyethyl Acrylate Crosspolymer | |
| Wasserstoffperoxid (35 Gew.-%ig, wässrig) | 18 |
| Natriumpyrophosphat | 0,02 |
| 4-Methyl-6-(2,4,4-trimethylpentyl)-1-hydroxy-2-pyridon | 0,002 |
| Propylenglykol | 1 |
| Wasser | ad 100 |

### Herstellung:

4-Methyl-6-(2,4,4 trimethylpentyl)-1-hydroxy-2-pyridon wird in Propylenglykol gelöst. Aristoflex^{®} TAC wird in Wasser aufgelöst, sodann wird die Wasserstoffperoxidlösung eingerührt, gefolgt vom Stabilisator Natriumpyrophosphat.

Es entsteht ein Gel mit einer Viskosität von ca. 3 000 mPa · s bei 20 °C.

### Färbeprozedur:

50 ml der Färbebasis werden mit 50 ml des Entwicklergels verrührt und auf das Haar appliziert. Nach 30 Minuten wird ausgespült.

### Formulierungsbeispiel 5: Fixiergel für Dauerwellen

| Inhaltsstoff | Gew.-% |
|---|---|
| Aristoflex^{®} TAC | 0,5 |
| Ammonium Acryloyldimethyltaurate/Carboxyethyl Acrylate Crosspolymer | |
| Wasserstoffperoxid (35 Gew.-%ig, wässrig) | 5 |
| Natriumpyrophosphat | 0,02 |
| 4-Methyl-6-(2,4,4-trimethylpentyl)-1-hydroxy-2-pyridon | 0,002 |
| Propylenglykol | 1 |
| Wasser | ad 100 |

### Herstellung:

4-Methyl-6-(2,4,4-trimethylpentyl)-1-hydroxy-2-pyridon wird in Proplenglykol gelöst.
Aristoflex^{®} TAC wird in Wasser gelöst, die Wasserstoffperoxidlösung und das Pyrophosphat sowie die Propylenglykollösung eingetragen und homogenisiert.

### Formulierungsbeispiel 6: Fixiergel für Dauerwellen

| Inhaltsstoff | Gew.-% |
|---|---|
| Aristoflex^{®} TAC | 0,5 |
| Ammonium Acryloyldimethyltaurate/Carboxyethyl Acrylate Crosspolymer | |
| Wasserstoffperoxid (35 Gew.-%ig, wässrig) | 5 |
| Natriumpyrophosphat | 0,02 |
| Polyquaternium-10 (Polymer IR-400) | 0,1 |
| 4-Methyl-6-(2,4,4-trimethylpentyl)-1-hydroxy-2-pyridon | 0,0005 |
| Propylenglykol | 1 |
| Wasser | ad 100 |

### Herstellung:

4-Methyl-6-(2,4,4-trimethylpentyl)-1-hydroxy-2-pyridon wird in Propylenglykol gelöst.
Polyquaternium-10 wird in Wasser gelöst, danach wird Aristoflex^{®} TAC unter Rühren zugegeben, die Wasserstoffperoxidlösung und das Pyrophosphat sowie die Propylenglykollösung eingetragen und homogenisiert.

### Formulierungsbeispiel 7: Vorwaschspray für Wäsche

| Inhaltsstoff | Gew.-% |
|---|---|
| Aristoflex^{®} TAC | 0,5 |
| Ammonium Acryloyldimethyltaurate/Carboxyethyl Acrylate Crosspolymer | |
| Wasserstoffperoxid (35 Gew.-%ig, wässrig) | 10 |
| Laureth-7 | 5 |
| Laureth-3 | 2 |
| Hostapur^{®} SAS 60 (Sodium C14-17 Alkyl Sec Sulfonate) | 5 |
| 4-Methyl-6-(2,4,4-trimethylpentyl)-1-hydroxy-2-pyridon | 0,0005 |
| Propylenglykol | 1 |
| Wasser | ad 100 |

### Herstellung:

4-Methyl-6-(2,4,4-trimethylpentyl)-1-hydroxy-2-pyridon wird in Propylenglykol gelöst.
Die Tenside werden in Wasser gelöst, danach wird Aristoflex^{®} TAC unter Rühren zugegeben, die Wasserstoffperoxidlösung sowie die Propylenglykollösung eingetragen und homogenisiert. Das Produkt wird auf pH = 4 eingestellt.

### Formulierungsbeispiel 8: Fleckentferner

| Inhaltsstoff | Gew.-% |
|---|---|
| Aristoflex^{®} TAC | 0,5 |
| Ammonium Acryloyldimethyltaurate/Carboxyethyl Acrylate Crosspolymer | |
| Wasserstoffperoxid (35 Gew.-%ig, wässrig) | 10 |
| Sodium C12-C14 Olefin Sulfonate | 5 |
| 4-Methyl-6-(2,4,4-trimethylpentyl)-1-hydroxy-2-pyridon | 0,0005 |
| Propylenglykol | 1 |
| Wasser | ad 100 |

### Herstellung:

4-Methyl-6-(2,4,4-trimethylpentyl)-1-hydroxy-2-pyridon wird in Propylenglykol gelöst.
Das Tensid wird in Wasser gelöst, danach wird Aristoflex^{®} TAC unter Rühren zugegeben, die Wasserstoffperoxidlösung sowie die Propylenglykollösung eingetragen und homogenisiert. Das Produkt wird auf pH = 7 eingestellt.

### Formulierungsbeispiel 9: Fleckentferner Gel

| Inhaltsstoff | Gew.-% |
|---|---|
| Aristoflex^{®} TAC | 0,5 |
| Ammonium Acryloyldimethyltaurate/Carboxyethyl Acrylate Crosspolymer | |
| Wasserstoffperoxid (35 Gew.-%ig, wässrig) | 12 |
| C 12/14 Pareth-7 | 7 |
| 4-Methyl-6-(2,4,4-trimethylpentyl)-1-hydroxy-2-pyridon | 0,0005 |
| Propylenglykol | 1 |
| MEA-Dodecylbenzolsulfonate | 6 |
| Wasser | ad 100 |

### Herstellung:

4-Methyl-6-(2,4,4-trimethylpentyl)-1-hydroxy-2-pyridon wird in Propylenglykol gelöst.

Die Tenside werden in Wasser gelöst, danach wird Aristoflex^{®} TAC unter Rühren zugegeben, die Wasserstoffperoxidlösung sowie die Propylenglykollösung eingetragen und homogenisiert. Das Produkt wird auf pH = 4,7 eingestellt.

## Patentansprüche

1. Zusammensetzung enthaltend
a) eine oder mehrere Substanzen ausgewählt aus der Gruppe bestehend aus Wasserstoffperoxid und Wasserstoffperoxid freisetzenden Substanzen,
b) Wasser,
c) ein oder mehrere Polymere mit verdickenden Eigenschaften und
d) eine oder mehrere Substanzen ausgewählt aus Verbindungen der Formel (I) und deren Salzen 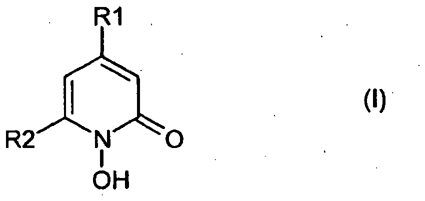 worin R1 H oder ein C₁-C₄ Alkylrest und R2 H, ein unsubstituierter oder mit Halogen substituierter, verzweigter oder unverzweigter C₁-C₂₀-Alkylrest, ein unsubstituierter oder mit Halogen substituierter C₅-C₈ Cycloalkylrest, ein unsubstituierter oder mit Halogen substituierter C₆-C₁₀ Arylrest oder ein unsubstituierter oder mit Halogen substituierter, verzweigter oder unverzweigter C₇-C₂₀ Aralkylrest ist.

2. Zusammensetzung nach Anspruch 1, **dadurch gekennzeichnet, dass** die eine oder die mehreren Substanzen der Komponente a) ausgewählt sind aus der Gruppe bestehend aus Wasserstoffperoxid, Harnstoffperoxid, Perboraten, Persulfaten und Mischungen davon.

3. Zusammensetzung nach Anspruch 1 oder 2, **dadurch gekennzeichnet, dass** die Substanz der Komponente a) Wasserstoffperoxid ist und das Wasserstoffperoxid in Mengen von 0,5 bis 20 Gew.-% in der Zusammensetzung enthalten ist, bezogen auf das Gesamtgewicht der Zusammensetzung.

4. Zusammensetzung nach einem oder mehreren der Ansprüche 1 bis 3, **dadurch gekennzeichnet, dass** das eine oder die mehreren Polymere mit verdickenden Eigenschaften der Komponente c) ausgewählt sind aus der Gruppe bestehend aus Polymeren mit C-C Backbone, Biopolymeren, modifizierten Biopolymeren, Assoziatiwerdickern und Polyalkylenglykolen.

5. Zusammensetzung nach Anspruch 4, **dadurch gekennzeichnet, dass** das eine oder die mehreren Polymere mit verdickenden Eigenschaften der Komponente c) ausgewählt sind aus Polymeren mit C-C-Backbone.

6. Zusammensetzung nach Anspruch 5, **dadurch gekennzeichnet, dass** das eine oder die mehreren Polymere mit C-C-Backbone ausgewählt sind aus teilweise oder vollständig neutralisierten, sulfonsäuregruppenhaltigen Polymeren.

7. Zusammensetzung nach Anspruch 6, **dadurch gekennzeichnet, dass** das eine oder die mehreren teilweise oder vollständig neutralisierten, sulfonsäuregruppenhaltigen Polymere ausgewählt sind aus der Gruppe bestehend aus Homo- oder Copolymeren von Acrylamidomethylpropansulfonsäue und deren Salzen.

8. Zusammensetzung nach Anspruch 4, **dadurch gekennzeichnet, dass** das eine oder die mehreren Polymere mit verdickenden Eigenschaften der Komponente c) ausgewählt sind aus der Gruppe bestehend aus Biopolymeren und modifizierten Biopolymeren.

9. Zusammensetzung nach Anspruch 4, **dadurch gekennzeichnet, dass** das eine oder die mehreren Polymere mit verdickenden Eigenschaften der Komponente c) ausgewählt sind aus Assoziatiwerdickern.

10. Zusammensetzung nach Anspruch 9, **dadurch gekennzeichnet, dass** der eine oder die mehreren Assoziativverdicker ausgewählt sind aus Phosphorsäureestern.

11. Zusammensetzung nach Anspruch 4, **dadurch gekennzeichnet, dass** das eine oder die mehreren Polymere mit verdickenden Eigenschaften der Komponente c) ausgewählt sind aus Polyalkylenglykolen.

12. Zusammensetzung nach einem oder mehreren der Ansprüche 1 bis 11, **dadurch gekennzeichnet, dass** die eine oder die mehreren Verbindungen der Komponente d) in Form der Säure oder in Form ihrer Alkali-, Erdalkali- oder Aminsalze oder ihrer Salze mit polymeren Gegenionen vorliegen.

13. Zusammensetzung nach einem oder mehreren der Ansprüche 1 bis 12, **dadurch gekennzeichnet, dass** in der einen oder in den mehreren Verbindungen der Formel (I) oder in ihren Salzen R1 Methyl ist und R2 Cyclohexyl oder 2,4,4-Trimethylpentyl ist.

14. Zusammensetzung nach einem oder mehreren der Ansprüche 1 bis 12, **dadurch gekennzeichnet, dass** R1 und R2 in der einen oder in den mehreren Verbindungen der Formel (I) oder in ihren Salzen H sind.

15. Zusammensetzung nach einem oder mehreren der Ansprüche 1 bis 14, **dadurch gekennzeichnet, dass** sie die eine oder die mehreren Substanzen der Komponente d) in Mengen von 0,1 ppm bis 2 Gew.-% enthält, bezogen auf das Gesamtgewicht der Zusammensetzung.

16. Zusammensetzung nach einem oder mehreren der Ansprüche 1 bis 15, **dadurch gekennzeichnet, dass** sie einen pH-Wert von 2 bis 10 aufweist.

17. Zusammensetzung nach einem oder mehreren der Ansprüche 1 bis 16, **dadurch gekennzeichnet, dass** sie eine Zusammensetzung zum Bleichen und/oder Färben von Haaren oder eine Dauerwellformulierung ist.

18. Zusammensetzung nach einem oder mehreren der Ansprüche 1 bis 16, **dadurch gekennzeichnet, dass** sie eine oxidative Reinigerformulierung ist.

19. Zusammensetzung nach einem oder mehreren der Ansprüche 1 bis 16, **dadurch gekennzeichnet, dass** sie ein Fleckengel zur Vorbehandlung von Wäsche, ein Vorwaschspray, ein Fleckentferner, ein Oberflächenreiniger, ein Toilettenreiniger, ein Multifunktionsfleckengel oder ein Bleichgel ist.

20. Verwendung einer oder mehrerer Substanzen ausgewählt aus der Gruppe bestehend aus Hydroxypyridonen und deren Salzen zur Stabilisierung eines oder mehrerer Polymere mit verdickenden Eigenschaften in einer Zusammensetzung nach einem oder mehreren der Ansprüche 1 bis 19.

21. Verwendung nach Anspruch 20, **dadurch gekennzeichnet, dass** das eine oder die mehreren Polymere mit verdickenden Eigenschaften ausgewählt sind aus teilweise oder vollständig neutralisierten, sulfonsäuregruppenhaltigen Polymeren.

22. Verwendung nach Anspruch 21, **dadurch gekennzeichnet, dass** das eine oder die mehreren teilweise oder vollständig neutralisierten, sulfonsäuregruppenhaltigen Polymere ausgewählt sind aus der Gruppe bestehend aus Homo- oder Copolymeren von Acrylamidomethylpropansulfonsäure und deren Salzen.

## Claims

1. A composition comprising
a) one or more substances selected from the group consisting of hydrogen peroxide and hydrogen peroxide-releasing substances,
b) water,
c) one or more polymers having thickening properties and
d) one or more substances selected from compounds of the formula (I) and salts thereof 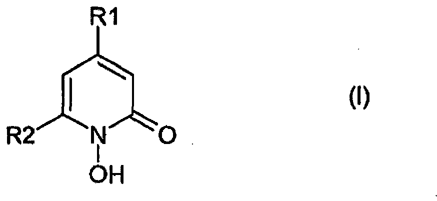 in which R1 is H or a C₁-C₄ alkyl radical and R2 is H, an unsubstituted or halogen-substituted, branched or unbranched C₁-C₂₀ alkyl radical, an unsubstituted or halogen-substituted C₅-C₈ cycloalkyl radical, an unsubstituted or halogen-substituted C₆-C₁₀ aryl radical or an unsubstituted or halogen-substituted, branched or unbranched C₇-C₂₀ aralkyl radical.

2. The composition as claimed in claim 1, wherein the one or more substances of component a) are selected from the group consisting of hydrogen peroxide, urea peroxide, perborates, persulfates and mixtures thereof.

3. The composition as claimed in claim 1 or 2, wherein the substance of component a) is hydrogen peroxide and the hydrogen peroxide is present in the composition in amounts of 0.5 to 20% by weight based on the total weight of the composition.

4. The composition as claimed in one or more of claims 1 to 3, wherein the one or more polymers having thickening properties of component c) are selected from the group consisting of polymers having a C-C backbone, biopolymers, modified biopolymers, associative thickeners and polyalkylene glycols.

5. The composition as claimed in claim 4, wherein the one or more polymers having thickening properties of component c) are selected from polymers having a C-C backbone.

6. The composition as claimed in claim 5, wherein the one or more polymers having a C-C backbone are selected from partly or fully neutralized polymers containing sulfo groups.

7. The composition as claimed in claim 6, wherein the one or more partly or fully neutralized polymers containing sulfo groups are selected from the group consisting of homo- or copolymers of acrylamidomethylpropanesulfonic acid and salts thereof.

8. The composition as claimed in claim 4, wherein the one or more polymers having thickening properties of component c) are selected from the group consisting of biopolymers and modified biopolymers.

9. The composition as claimed in claim 4, wherein the one or more polymers having thickening properties of component c) are selected from associative thickeners.

10. The composition as claimed in claim 9, wherein the one or more associative thickeners are selected from phosphoric esters.

11. The composition as claimed in claim 4, wherein the one or more polymers having thickening properties of component c) are selected from polyalkylene glycols.

12. The composition as claimed in one or more of claims 1 to 11, wherein the one or more compounds of component d) are present in the form of the acid or in the form of the alkali metal, alkaline earth metal or amine salts thereof or salts thereof with polymeric counterions.

13. The composition as claimed in one or more of claims 1 to 12, wherein, in the one or more compounds of the formula (I) or in the salts thereof, R1 is methyl and R2 is cyclohexyl or 2,4,4-trimethylpentyl.

14. The composition as claimed in one or more of claims 1 to 12, wherein R1 and R2 in the one or more compounds of the formula (I) or in the salts thereof are each H.

15. The composition as claimed in one or more of claims 1 to 14, which comprises the one or more substances of component d) in amounts of 0.1 ppm to 2% by weight, based on the total weight of the composition.

16. The composition as claimed in one or more of claims 1 to 15, which has a pH of 2 to 10.

17. The composition as claimed in one or more of claims 1 to 16, which is a composition for bleaching and/or coloring of hair or a permanent wave formulation.

18. The composition as claimed in one or more of claims 1 to 16, which is an oxidative cleanser formulation.

19. The composition as claimed in one or more of claims 1 to 16, which is a stain removal gel for pretreatment of laundry, a prewash spray, a stain remover, a surface cleaner, a toilet cleaner, a multifunctional stain removal gel or a bleaching gel.

20. The use of one or more substances selected from the group consisting of hydroxypyridones and salts thereof for stabilization of one or more polymers having thickening properties in a composition as claimed in one or more of claims 1 to 19.

21. The use as claimed in claim 20, wherein the one or more polymers having thickening properties are selected from partly or fully neutralized polymers containing sulfo groups.

22. The use as claimed in claim 21, wherein the one or more partly or fully neutralized polymers containing sulfo groups are selected from the group consisting of homo- or copolymers of acrylamidomethylpropanesulfonic acid and salt thereof.

## Revendications

1. Composition contenant
a) une ou plusieurs substances choisies dans le groupe constitué par le peroxyde d'hydrogène et les substances libérant du peroxyde d'hydrogène,
b) de l'eau,
c) un ou plusieurs polymères dotés de propriétés épaississantes et
d) une ou plusieurs substances choisies parmi les composés de formule (I) et leurs sels 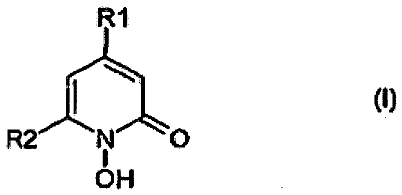 dans laquelle R1 représente H ou un radical C₁-C₄-alkyle et R2 représente H, un radical C₁-C₂₀-alkyle ramifié ou non ramifié, non substitué ou substitué par halogène, un radical C₅-C₈-cycloalkyle non substitué ou substitué par halogène, un radical C₆-C₁₀-aryle non substitué ou substitué par halogène ou un radical C₇-C₂₀-aralkyle ramifié ou non ramifié, non substitué ou substitué par halogène.

2. Composition selon la revendication 1, **caractérisée en ce que** ladite une ou lesdites plusieurs substances du composant a) sont choisies dans le groupe constitué par le peroxyde d'hydrogène, le peroxyde d'urée, les perborates, les persulfates et leurs mélanges.

3. Composition selon la revendication 1 ou 2, **caractérisée en ce que** la substance du composant a) est le peroxyde d'hydrogène et le peroxyde d'hydrogène est contenu en des quantités de 0,5 à 20% en poids dans la composition, par rapport au poids total de la composition.

4. Composition selon l'une ou plusieurs des revendications 1 à 3, **caractérisée en ce que** ledit un ou lesdits plusieurs polymères dotés de propriétés épaississantes du composant c) sont choisis dans le groupe constitué par les polymères présentant une épine dorsale C-C, les biopolymères, les biopolymères modifiés, les épaississants associatifs et les polyalkylèneglycols.

5. Composition selon la revendication 4, **caractérisée en ce que** ledit un ou lesdits plusieurs polymères dotés de propriétés épaississantes du composant c) sont choisis parmi les polymères présentant une épine dorsale C-C.

6. Composition selon la revendication 5, **caractérisée en ce que** ledit un ou lesdits plusieurs polymères présentant une épine dorsale C-C sont choisis parmi les polymères contenant des groupes acide sulfonique, partiellement ou totalement neutralisés.

7. Composition selon la revendication 6, **caractérisée en ce que** ledit un ou lesdits plusieurs polymères contenant des groupes acide sulfonique, partiellement ou totalement neutralisés sont choisis dans le groupe constitué par les homopolymères et les copolymères de l'acide acrylamidométhylpropanesulfonique et leurs sels.

8. Composition selon la revendication 4, **caractérisée en ce que** ledit un ou lesdits plusieurs polymères dotés de propriétés épaississantes du composant c) sont choisis dans le groupe constitué par les biopolymères et les biopolymères modifiés.

9. Composition selon la revendication 4, **caractérisée en ce que** ledit un ou lesdits plusieurs polymères dotés de propriétés épaississantes du composant c) sont choisis parmi les épaississants associatifs.

10. Composition selon la revendication 9, **caractérisée en ce que** ledit un ou lesdits plusieurs épaississants associatifs sont choisis parmi les esters de l'acide phosphorique.

11. Composition selon la revendication 4, **caractérisée en ce que** ledit un ou lesdits plusieurs polymères dotés de propriétés épaississantes du composant c) sont choisis parmi les polyalkylèneglycols.

12. Composition selon l'une ou plusieurs des revendications 1 à 11, **caractérisée en ce que** ledit un ou lesdits plusieurs composés du composant d) se trouvent sous forme acide ou sous forme de leurs sels de métal alcalin, alcalino-terreux ou d'amine ou de leurs sels avec des contre-ions polymères.

13. Composition selon l'une ou plusieurs des revendications 1 à 12, **caractérisée en ce que** dans ledit un ou lesdits plusieurs composés de formule (I) ou dans leurs sels, R1 représente méthyle et R2 représente cyclohexyle ou 2,4,4-triméthylpentyle.

14. Composition selon l'une ou plusieurs des revendications 1 à 12, **caractérisée en ce que** R1 et R2 dans ledit un ou lesdits plusieurs composés de formule (I) ou dans leurs sels représentent H.

15. Composition selon l'une ou plusieurs des revendications 1 à 14, **caractérisée en ce qu'**elle contient ladite une ou lesdites plusieurs substances du composant d) en des quantités de 0,1 ppm à 2% en poids, par rapport au poids total de la composition.

16. Composition selon l'une ou plusieurs des revendications 1 à 15, **caractérisée en ce qu'**elle présente un pH de 2 à 10.

17. Composition selon l'une ou plusieurs des revendications 1 à 16, **caractérisée en ce qu'**il s'agit d'une composition pour l'éclaircissement et/ou la coloration de cheveux ou d'une formulation pour permanente.

18. Composition selon l'une ou plusieurs des revendications 1 à 16, **caractérisée en ce qu'**il s'agit d'une formulation d'agent de nettoyage oxydante.

19. Composition selon l'une ou plusieurs des revendications 1 à 16, **caractérisée en ce qu'**il s'agit d'un gel pour taches destiné au prétraitement de linge, d'un spray de prélavage, d'un détachant, d'un agent de nettoyage de surfaces, d'un agent de nettoyage de toilettes, d'un gel pour taches multifonction ou d'un gel de blanchiment.

20. Utilisation d'une ou de plusieurs substances choisies dans le groupe constitué par les hydroxypyridones et leurs sels pour la stabilisation d'un ou de plusieurs polymères dotés de propriétés épaississantes dans une composition selon l'une ou plusieurs des revendications 1 à 19.

21. Utilisation selon la revendication 20, **caractérisée en ce que** ledit un ou lesdits plusieurs polymères dotés de propriétés épaississantes sont choisis parmi les polymères contenant des groupes acide sulfonique, partiellement ou totalement neutralisés.

22. Utilisation selon la revendication 21, **caractérisée en ce que** ledit un ou lesdits plusieurs polymères contenant des groupes acide sulfonique, partiellement ou totalement neutralisés sont choisis dans le groupe constitué par les homopolymères et les copolymères de l'acide acrylamidométhylpropanesulfonique et leurs sels.
